# FASCICULE DE BREVET EUROPEEN

(11) **EP 0 648 203 B1**
(45) Date de publication et mention de la délivrance du brevet: **18.11.1998**
(21) Numéro de dépôt: 94910443.4
(22) Date de dépôt: 21.03.1994
(51) Int. Cl.: C07C 237/46, A61K 49/04

(54) **NOUVEAUX COMPOSES POLYIODES, LEUR PREPARATION ET LEUR UTILISATION EN TANT QUE PRODUITS DE CONTRASTE POUR LA RADIOLOGIE**
POLYIODIERTE VERBINDUNGEN, IHRE HERSTELLUNG UND IHRE VERWENDUNG ALS KONTRASTMITTEL IN DER RADIOLOGIE
NOVEL POLYIODINATED COMPOUNDS, PREPARATION THEREOF AND USE THEREOF AS RADIOLOGICAL CONTRAST AGENTS

(30) Priorité: 22.03.1993 FR 9303269
(43) Date de publication de la demande: 19.04.1995
(73) Titulaire: GUERBET S.A., F-93420 Villepinte (FR)
(72) Inventeur: MEYER, Dominique, F-94100 Saint-Maur (FR); LE GRENEUR, Soizic, F-91440 Bures-sur-Yvette (FR); LE LEM, Gael, F-92150 Suresnes (FR); SIMONOT, Christian, F-75020 Paris (FR); CHAMBON, Catherine, F-91430 Igny (FR)
(74) Mandataire: Le Guen, Gérard
(86) Numéro de dépôt international: FR9400305
(87) Numéro de publication internationale: WO9421600

(56) Documents cités:
- EP-A- 0 074 307
- EP-A- 0 354 836
- EP-A- 0 436 316
- DE-A- 2 039 214
- FR-A- 2 104 739
- FR-A- 2 272 640
- GB-A- 1 346 795
- Investigative Radiology, 1994, Vol. 29, Suppl. 2, S271-S274

## Description

La présente invention concerne des composés utilisables en tant que produits de contraste pour la radiologie par rayons X.

La présente invention concerne plus particulièrement des composés utilisables en tant que produits de contraste possédant une rémanence vasculaire.

Il existe toujours à l'heure actuelle un besoin pour ce type de produits.

En effet, les produits classiques ioniques ou non ioniques habituellement utilisés tels que les dérivés triiodés de l'acide isophtalique sont extravasés dans le compartiment interstitiel et excrétés par voie rénale très rapidement. Le temps disponible pour obtenir une image du compartiment vasculaire de qualité suffisante est ainsi très réduit.

Pour pallier cet inconvénient, on a proposé de réitérer les injections de produits de contraste classiques. Cette solution entraîne cependant une augmentation des doses et un risque d'effets secondaires indésirables plus élevé. Elle a pour conséquence également un coût des examens du compartiment vasculaire plus important.

Des produits de contraste à rémanence vasculaire pour la radiologie par rayons X ont été par ailleurs proposés, ils sont tous basés sur le principe de l'accrochage d'un certain nombre de molécules iodées sur un polymère de poids moléculaire élevé.

De cette façon, le produit de contraste est excrété lentement par filtration glomérulaire et reste confiné dans le compartiment vasculaire, au moins pendant un certain temps.

On peut citer à cet égard EP-354 836 et EP-344 202 décrivant des composés possédant un squelette de type dextran sur lequel sont greffés des groupes phényles iodés, EP 436316 décrivant des composés dont le squelette est de type polyacrylamide sur lequel sont greffés des groupes phényles iodés ou encore US 5 019 370 décrivant des composés à squelette polyacrylate ou polyamide.

L'inconvénient majeur des produits de l'art antérieur réside cependant dans la polydispersité du poids moléculaire des polymères utilisés, tel que le dextran ou la polylysine, ou à la copolymérisation des monomères de type acrylamide utilisés.

Cette polydispersité présente plusieurs inconvénients : tout d'abord, les composants du polymère iodé de bas poids moléculaire sont excrétés rapidement et ne contribuent plus à la concentration utile pour l'obtention de l'image vasculaire.

En outre, l'extravasation des polymères de bas poids moléculaire dans le compartiment interstitiel diminue le contraste à l'interface entre les compartiments vasculaire et interstitiel, et conduit à une image de qualité insuffisante.

Enfin, les composants du polymère iodé de haut poids moléculaire ne sont éliminés que très lentement par voie rénale ou par captation par le système réticuloendothélial et peuvent générer des réactions anaphylactoïdes indésirables.

Les composés proposés jusqu'à présent ne permettent donc pas de maîtriser de façon précise le confinement d'un produit de contraste dans le compartiment vasculaire, ni sa biocompatibilité.

On connaît par ailleurs de FR 2 272 640, des composés polyiodés, comprenant jusqu'à 3 cycles phényle triiodés, réunis 2 à 2 par des ponts alkylènediamido; FR 2 104 739 décrit des dimères et trimères de l'acide diamino-3,5-triiodo-2,4,6-benzoïque, réunis 2 à 2 par des ponts amino alkylène; DE 2039214 et GB 1346795 décrivent des dérivés triphényles iodés, chacun des noyaux phényles étant lié à un atome d'azote central par un pont alkylèneamido.

Ces composés ne sont cependant pas décrits comme susceptibles de présenter une rémanence vasculaire.

La présente invention a pour but de fournir des composés utilisables comme produits de contraste pour la radiologie par rayons X qui ne possèdent pas les inconvénients des composés de l'état de la technique tout en possédant une bonne rémanence vasculaire.

L'invention a pour objet des composés polyiodés d'un poids moléculaire unique possédant une concentration moléculaire en iode supérieure à environ 20 % en poids, notamment supérieure à environ 30 % en poids, comportant au moins 9 atomes d'iode et possédant un poids moléculaire supérieur à 2000 et inférieur à environ 50.000, notamment supérieur à 2000 et inférieur à environ 20.000, caractérisés en ce qu'ils possèdent soit une charge électrique globale nulle, soit au moins deux charges anioniques et en ce qu'ils persistent dans le compartiment vasculaire à une valeur égale à au moins environ 30 % en poids de la dose injectée chez un sujet cinq minutes après l'administration intra-vasculaire chez ledit sujet.

Par poids moléculaire unique, on entend dans le cadre de la présente invention, un poids moléculaire qui n'est pas défini comme une moyenne des différents poids moléculaires présents pour le même composé, par opposition à la dispersité des poids moléculaires des polymères de l'état de la technique cités dans ce qui précède.

En d'autres termes, un composé selon l'invention possédant un poids moléculaire unique s'entend d'un produit présentant un indice de polydispersité égal à 1.

L'invention a en particulier pour objet des composés polyiodés ayant un poids moléculaire supérieur à 2000 et inférieur à 50.000 de formule générale I:

A―(―X)ₘ (I)

dans laquelle :
- A représente le reste d'une molécule polyfonctionnelle comportant un atome de carbone central tri- ou tétra-substitué, un atome central d'azote ou de phosphore tri-substitué ou un groupe P=O tri-substitué et/ou au moins un cycle carboné aromatique ou non, comportant éventuellement un ou plusieurs atomes d'iode, ou au moins un hétérocycle aromatique ou non aromatique comportant de 1 à 4 hétéroatomes choisis parmi O, S, N et P, sur laquelle sont fixés les m groupes X par l'intermédiaire de m liaisons ou de m groupes choisis parmi -CO-, >N-R₅, -O-, >NCOR₅, avec R₅ représentant H, un groupe alkyle linéaire ou ramifié en C₁-C₁₀, hydroxy- ou polyhydroxyalkyle linéaire ou ramifié en C₁-C₁₀, alkoxy (en C₁-C₅)alkyle linéaire ou ramifié en C₁-C₁₀, hydroxy- ou polyhydroxyalkoxy en (C₁-C₅) alkyle linéaire ou ramifié en C₁-C₁₀;
- les groupes X, identiques ou différents, représentent: 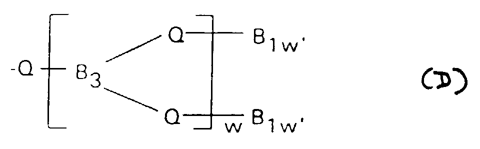 avec w = Σⁿₒ2ⁿ et w' = 2ⁿ, n représentant un entier de 0 à 4,
   et les groupes Q, identiques ou différents entre eux, représentent une liaison simple ou un groupe choisi parmi: 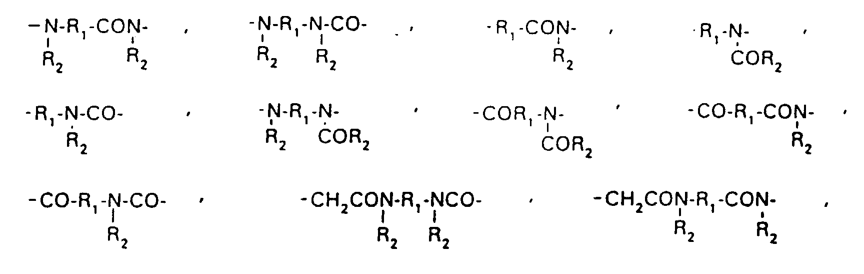 dans lesquels R₁ est choisi parmi une liaison simple et les groupes alkylène à chaîne linéaire ou ramifiée en C₁-C₁₀, hydroxy- ou polyhydroxy- alkylène à chaîne linéaire ou ramifiée en C₁-C₁₀, alkoxy (en C₁-C₅) alkylène à chaîne linéaire ou ramifiée en C₁-C₁₀, hydroxy- ou polyhydroxy- alkoxy (en C₁-C₅) alkylène à chaîne linéaire ou ramifiée, la chaîne alkylène étant éventuellement interrompue par un ou des atomes d'oxygène, les groupes R₂, identiques ou différents, sont choisis parmi H, les groupes alkyle à chaîne linéaire ou ramifiée en C₁-C₁₀, hydroxy- ou polyhydroxy- alkyle à chaîne linéaire ou ramifiée en C₁-C₁₀, alkoxy (en C₁-C₅) alkyle à chaîne linéaire ou ramifiée en C₁-C₁₀, hydroxy- ou poly-hydroxy- alkoxy (en C₁-C₅) alkyle à chaîne linéaire ou ramifiée;
   et les groupes B₁, identiques ou différents entre eux, sont représentés par les formules: 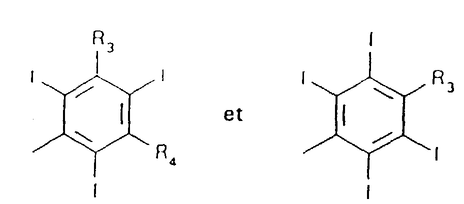 dans lesquelles R₃ est choisi parmi les groupes -COO⁻M⁺, avec M⁺ représentant H⁺ ou le cation physiologiquement acceptable d'une base organique ou minérale, 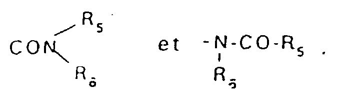 dans lesquels R₅ et R₆, identiques ou différents entre eux, sont définis comme précédemment pour R₅ ou R₅ et R₆ forment ensemble un groupe alkylène en C₄-C₈, hydroxyalkylène en C₄-C₈ ou polyhydroxyalkylène en C₄-C₈, à chaîne linéaire ou ramifiée éventuellement interrompue par un ou plusieurs atomes choisis parmi S, O, P et N, de sorte que R₅ et R₆ forment avec l'atome d'azote auquel ils sont liés un hétérocycle azoté de 5 à 12 chaînons éventuellement substitué par un ou plusieurs groupes hydroxy, hydroxy- ou polyhydroxyalkyle en C₁-C₄ à chaîne linéaire ou ramifiée et renfermant éventuellement un ou plusieurs hétéroatomes supplémentaires choisis parmi S, O, P et N, R₄ représente un groupe choisi parmi R₃ et Q-B₄, Q étant tel que défini précédemment et B₄ représentant un groupe : 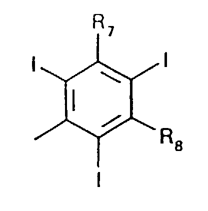 dans lequel R₇ et R₈, identiques ou différents entre eux, représentent R₃,
   et les groupes B₃, identiques ou différents entre eux, sont choisis parmi: 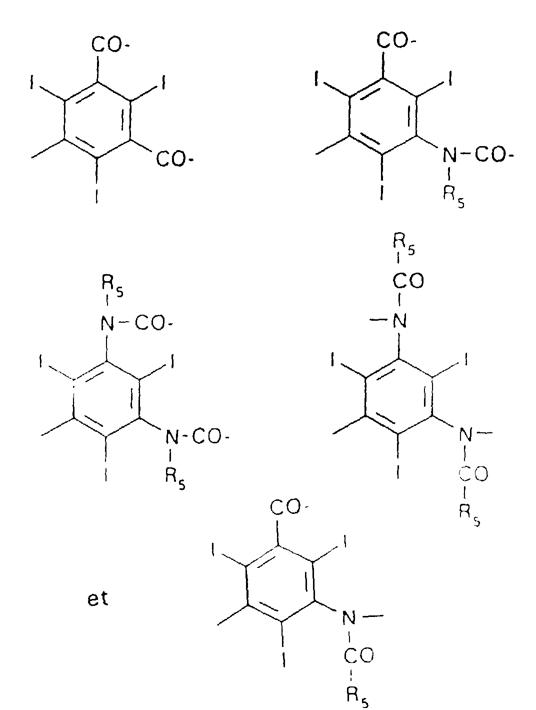 dans lesquels R₅ a les significations données ci-dessus
   et m représente un nombre entier de 2 à 12.

Avantageusement, l'invention a pour objet des composés de formule générale I telle que décrite ci-dessus, dans laquelle :
A représente un reste d'une molécule polyfonctionnelle comportant un atome de carbone central choisie parmi un reste de formule CR₉(R₁₀-Y-)₃- dans laquelle R₉ représente un atome d'hydrogène, un groupe alkyle en C₁-C₆ ou aryle en C₅-C₁₀ ou le groupe (R₁₀-Y), R₁₀ étant choisi parmi un groupe alkylène (en C₁-C₆), arylène (en C₅-C₁₀), alkyl (en C₁-C₁₀)arylène (en C₅-C₁₀), et aryl(en C₅-C₁₀)alkylène en (C₁-C₆), les groupes alkylène pouvant éventuellement être interrompus par un ou plus atomes d'oxygène, et les groupes alkyle, alkylène, aryle ou arylène étant éventuellement substitués par un ou plusieurs groupes OH,
Y étant choisi parmi un groupe -O-, -CO- ou 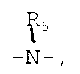 〉NCOR₅, R₅ étant tel que défini ci-dessus,
ou A représente un reste cycloalcane en C₅ à C₁₂, éventuellement polycyclique, comportant éventuellement de 1 à 6 atomes d'iode et de 2 à 12 substituants identiques ou différents de formule -R₁₁-(Y)_{q}, q représentant un entier de 1 à 3, R₁₁ étant une liaison simple ou un groupe alkylène linéaire ou ramifié en C₁-C₆ éventuellement substitué par un ou plusieurs groupes OH et/ou interrompu par un ou plusieurs atomes d'oxygène et Y étant tel que défini ci-dessus,
ou A représente un reste aromatique hydrocarboné en C₅-C₁₂ monocyclique ou bicyclique comportant éventuellement de 3 à 6 atomes d'iode et éventuellement un ou plusieurs substituants choisis parmi OH, NH₂, alkyle en C₁-C₆, hydroxy- ou polyhydroxyalkyle en C₁-C₆, COOH, COOR₁₂, -CO-NHR₁₂ ou -NR₆COR₅, R₁₂ représentant un groupe alkyle en C₁-C₄ et R₅ et R₆, identiques ou différents entre eux, représentent H, un groupe alkyle linéaire ou ramifié en C₁-C₁₀, hydroxy- ou polyhydroxyalkyle linéaire ou ramifié en C₁-C₁₀, alkoxy en (C₁-C₅) alkyle linéaire ou ramifié en C₁-C₁₀, hydroxy- ou polyhydroxy- alkoxy en (C₁-C₅) alkyle linéaire ou ramifié en C₁-C₁₀, ou R₅ et R₆ forment ensemble un groupe alkylène en C₄-C₈, hydroxyalkylène en C₄-C₈, ou polyhydroxyalkylène en C₄-C₈, à chaîne linéaire ou ramifiée éventuellement interrompue par un ou plusieurs atomes choisis parmi S, O, P et N, de sorte que R₅ et R₆ forment avec l'atome d'azote auquel ils sont liés un hétérocycle azoté de 5 à 12 chaînons éventuellement substitué par un ou plusieurs groupes hydroxy, hydroxy- ou polyhydroxyalkyle en C₁-C₄ à chaîne linéaire ou ramifiée et renfermant éventuellement un ou plusieurs hétéroatomes supplémentaires choisis parmi S, O, P et N, ce reste comportant de 2 à 12 substituants de formule - (R₁₁-(Y-)_{q}, Y, q et R₁₁ étant tels que définis précédemment;
ou A représente un reste hétérocyclique éventuellement aromatique, monocyclique ou bicyclique comportant de 5 à 10 chaînons dont 1 à 4 hétéroatomes choisis parmi O, S, N et P éventuellement substitué par 3 à 6 substituants identiques ou différents choisis parmi =O, et comportant de 3 à 12 substituants de formule -R₁₁-(-Y-)_{q}, R₁₁, Y et q étant tels que définis précédemment;
ou A représente un reste éventuellement cyclique comportant de 2 à 18 cycles aromatiques ou hétérocycliques tels que définis ci-dessus, liés entre eux par des groupes -R₁₁-, -OR₁₁-, ce reste comportant de 3 à 12 substituants de formule -R₁₁-(-Y)_{q}, R₁₁, Y et q étant tels que définis ci-dessus.

Des significations particulières de A sont les suivantes :
(i) - un reste de formule C(R₁₀-Y―)₄, R₁₀ représentant le groupe méthylène, phénylène, phénylméthylène ou le groupe -CH₂-O-CH₂- ou -CH₂-O-(CH₂)₂-, et Y étant tel que défini à la revendication 2 ;
(ii) - un reste de cycloalcane choisi parmi le cyclohexane et l'adamantane, substitués par 3 ou 4 substituants R₁₁-(Y-)_{q} tels que définis à la revendication 2 ;
(iii) - un groupe phényle éventuellement substitué par 3 à 4 atomes d'iode et un groupe biphényle éventuellement substitué par 4 à 6 atomes d'iode et comportant éventuellement de 3 à 6 substituants de formule R₁₁-(Y-)_{q}, R₁₁ et Y étant tels que définis à la revendication 2 et q représentant 1 à 4 ;
(iv) - un reste hétérocyclique constitué par le groupe 2,4,6-trioxo-1,3,5-triazine substitué sur les atomes d'azote par 3 substituants de formule R₁₁-(Y-)_{q} telle que définie à la revendication 2;
(v) - un macrocycle composé de 6 à 8 résidus phényle chacun substitué par un ou plusieurs substituants choisis parmi OH, NH₂, alkyle en C₁-C₆, hydroxy- ou polyhydroxyalkyle en C₁-C₆, COOH, COOR₁₂, -CO-NHR₁₂ ou - NR₆COR₅, R₁₂ représentant un groupe alkyle en C₁-C₄, et un ou plusieurs groupes de formule R₁₁-(Y-)_{q} telle que définie ci-dessus avec q = 1, les résidus phényle étant liés entre eux par un groupe méthylène, ou une cyclodextrine composée de 6 à 8 résidus glucosyle liés entre eux par une liaison osidique, un ou plusieurs groupes OH desdits résidus glucosyle étant éventuellement substitués par un reste carboxyméthyle, un ou plusieurs groupes OH desdits résidus glucosyle étant remplacés par un groupe -O- ou un macrocycle polyazoté ou un dérivé d'un acide polyaminocarboxylique.

Les produits de l'invention dans lesquels le groupe X est un groupe (D) correspondent par exemple à la formule ci-après :
avec n = 2, w = 7 et w' = 4, on obtient le composé :

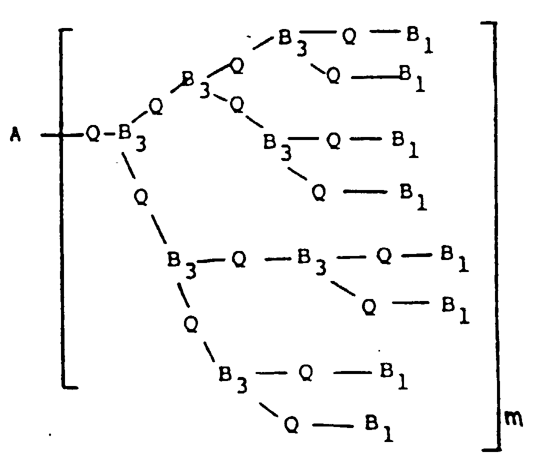

Il va de soi que l'invention englobe non seulement des composés de formule (I) sous forme de mélange racémique, mais également des stéréoisomères tels que énantiomères, diastéréoisomères, atropoisomères, isomères SYN-ANTI, ENDO-EXO, E-Z, liés à la présence d'atomes de carbone asymétriques et/ou aux empêchements de rotation dûs à l'encombrement stérique apporté par les atomes d'iode et/ou les substituants R des composés de l'invention.

Parmi les différents types de groupe A, on peut citer :
A) un reste de formule C(R₁₀-Y―)₄, R₁₀ représentant un groupe (CH₂)ₙ₁ avec n₁ = 1 à 5, éventuellement interrompu par 1 à 4 atomes d'oxygène, notamment le groupe méthylène ou le groupe -CH₂-O-CH₂- ou -CH₂-O-(CH₂)₂, un groupe phénylène ou phénylméthylène, et Y étant tel que défini précédemment,
   et plus particulièrement les groupes suivants : 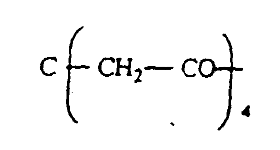 décrit dans J. Chem. Soc. (1922), 1638;

   C-(CH₂-O-CH₂-CH₂-CO)₄- ;

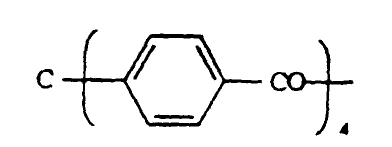

   décrit dans Angew. Chem. Int. Ed. Eng., 25, 1097, 1986 ; 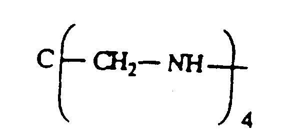 décrit dans US-3 994 972 J.C.S., 1938,1588-1595 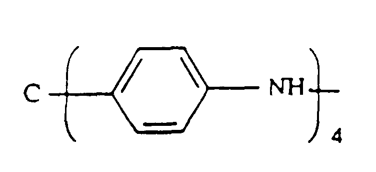 décrit dans F.A. Neugebauer, Chem. Ber., 109, 2389, 1976 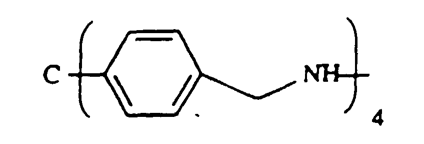 décrit dans Angew. Chem. Int. Ed. Engl. 25, 1097, 1986 et JACS, 101, 2728, 1979.
B) Un reste de cycloalcane choisi parmi le cyclohexane et l'adamantane, substitués par 3 ou 4 substituants R₁₁-(Y―)_{q} tels que définis ci-dessus et notamment les groupes suivants : 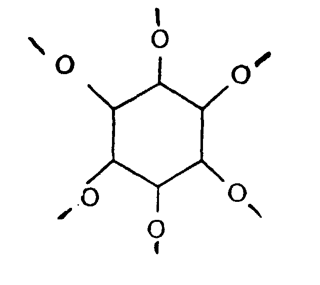 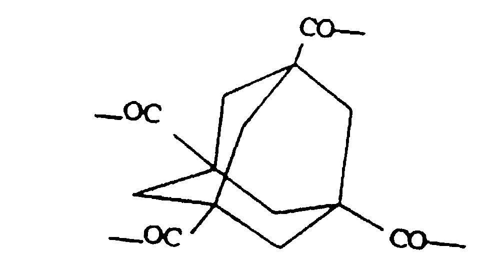 décrit dans Newkome, J. Org. Chem., 57, 358, 1992
C) un groupe phényle éventuellement substitué par 3 à 4 atomes d'iode ou un groupe biphényle éventuellement substitué par 4 à 6 atomes d'iode et comportant éventuellement de 3 à 6 substituants de formule -R₁₁―(Y―)_{q}, R₁₁ et Y étant tels que définis ci-dessus, notamment les groupes suivants : 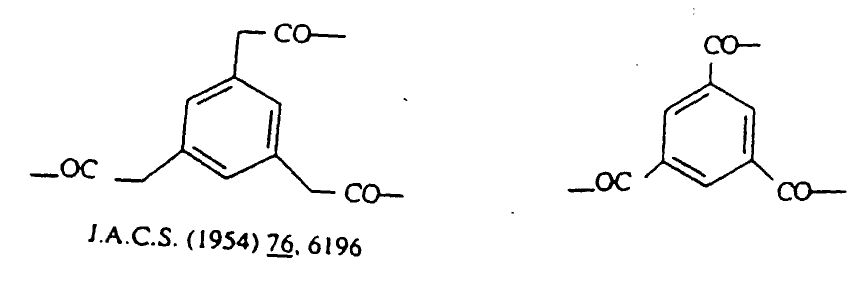 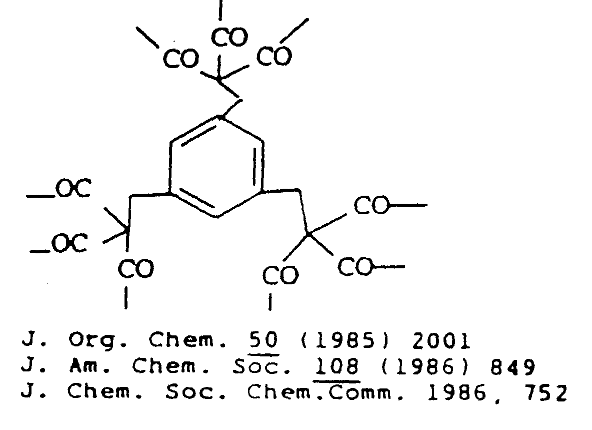 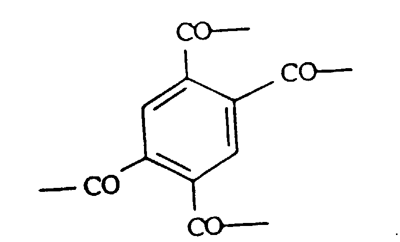 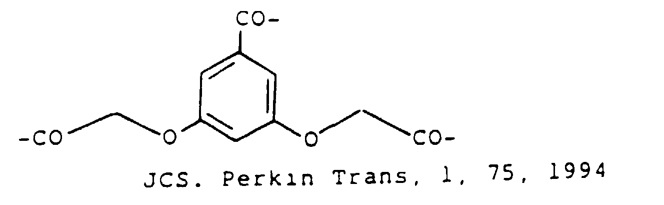 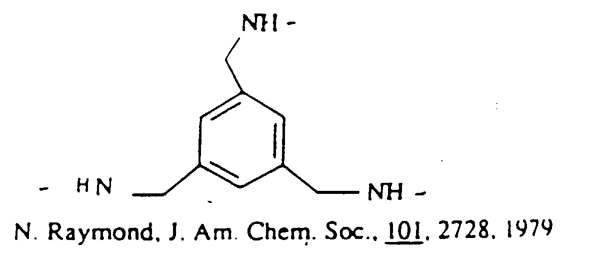 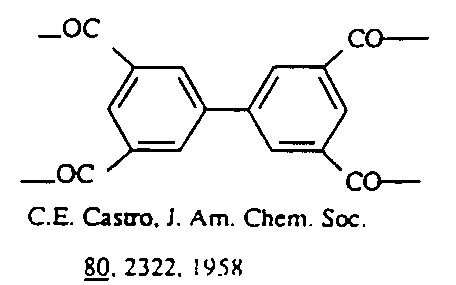 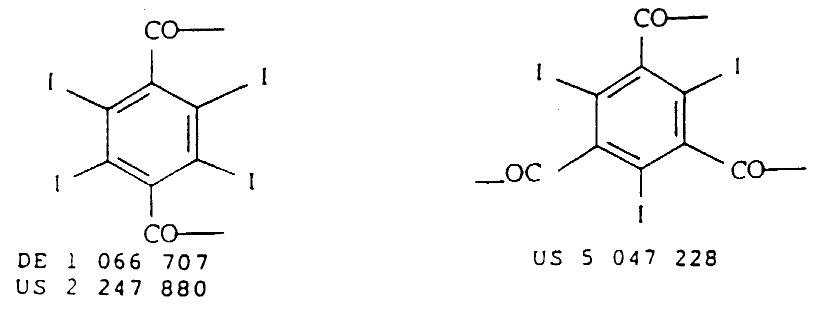 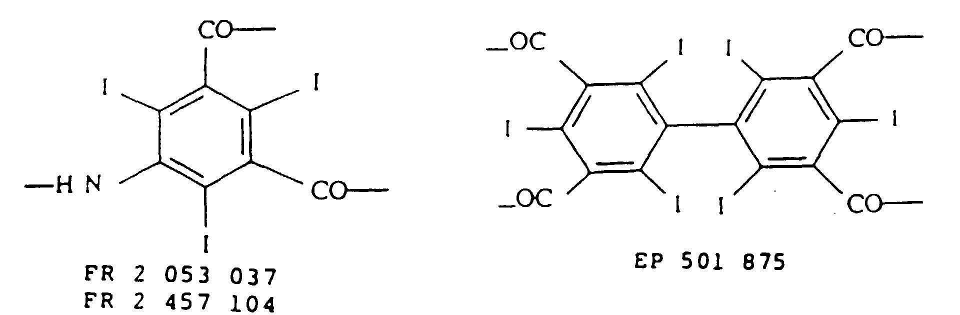 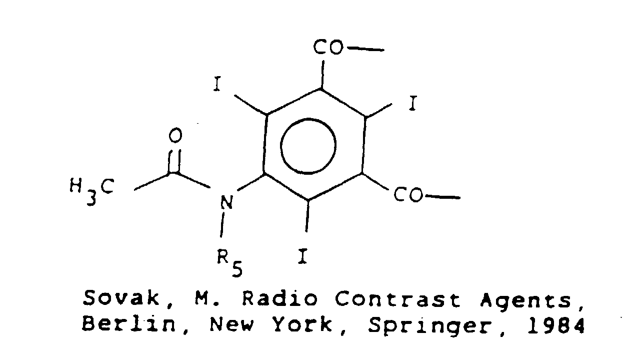
D) un reste hétérocyclique constitué par le groupe 2,4,6-trioxo-1,3,5-triazine substitué sur les atomes d'azote par 3 substituants de formule : -R₁₁―(Y₁―)_{q} telle que définie ci-dessus et notamment les groupes : 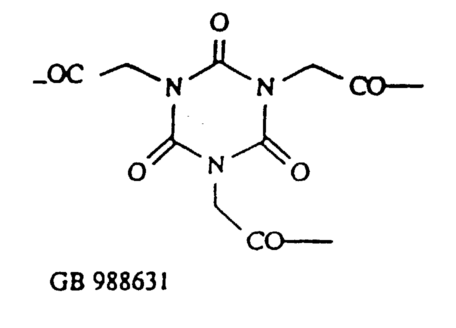 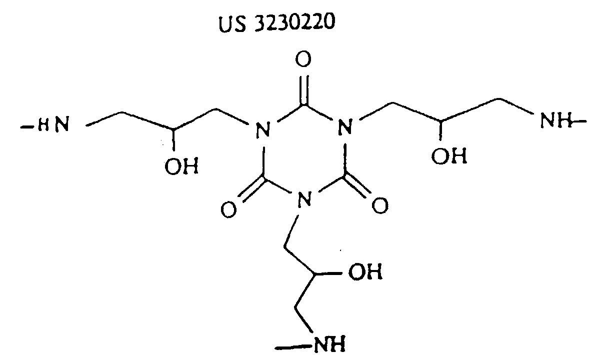
E) un macrocycle composé de 6 à 8 résidus phényle chacun substitué par un ou plusieurs substituants choisis parmi OH, NH₂, alkyle en C₁-C₆, hydroxy- ou polyhydroxyalkyle en C₁-C₆, COOH, COOR₁₂, -CO-NHR₁₂- ou - NR₆COR₅, R₁₂ représentant un groupe alkyle en C₁-C₄, et un ou plusieurs groupes de formule R₁₁―(Y―)_{q} telle que définie ci-dessus avec q = 1, les résidus phényle étant liés entre eux par un groupe méthylène, ou une cyclodextrine composée de 6 à 8 résidus glucosyle liés entre eux par une liaison osidique, un ou plusieurs groupes OH desdits résidus glucosyle étant éventuellement substitués par un reste carboxyméthyle, un ou plusieurs groupes OH desdits résidus glucosyle étant remplacés par un groupe -O- ou un macrocyle polyazoté tel que le 1,4,7-10-tétraazacyclododécane ou le 1-oxo-4,7,10-triazacyclododécane ou un dérivé d'un acide polyaminocarboxylique tel que le DOTA (acide 1,4,7,10-tétraazacyclododécane tétraacétique) ou le DO3A (acide 1,4,7-10-tétraazacyclododécane triacétique), notamment les restes :
   - dérivés des calixarènes à huit cycles de formule : 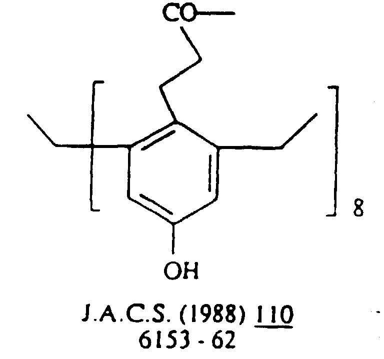
   - les restes dérivés de cyclodextrines α, β, γ et des carboxyméthylcyclodextrines α, β et γ (US 4 247 535, Carbohydr. Res. 63, 13(1978)), par suppression d'un ou plusieurs atomes d'H sur les restes glucosyle;
   - le DOTA, le DO3A.

Le groupe B₁ est avantageusement choisi parmi:

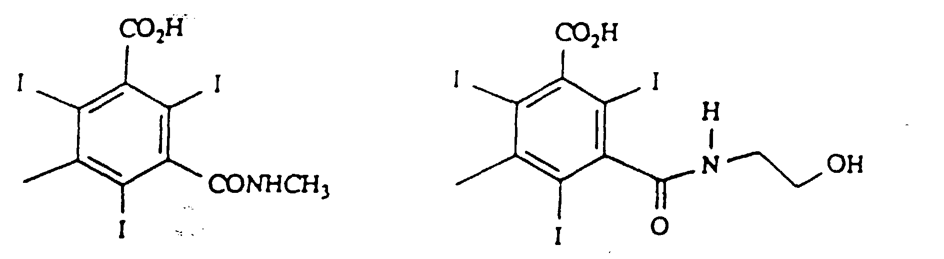

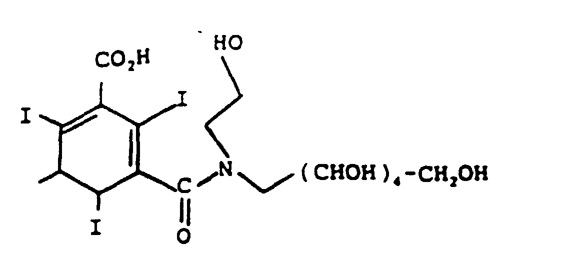

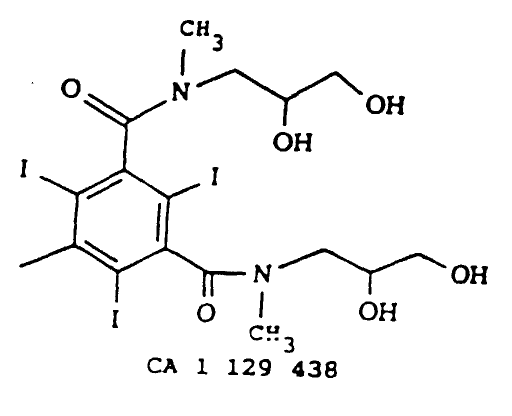

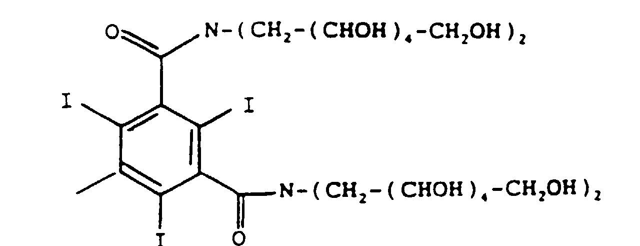

Le groupe B₃ est avantageusement choisi parmi:

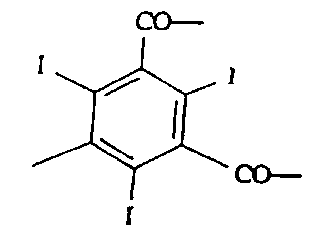

Le groupe Q est avantageusement choisi parmi:

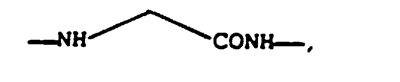

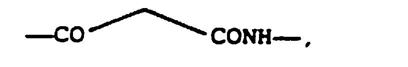

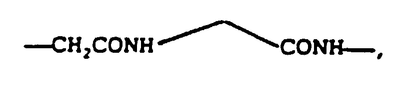

―(CH₂)ᵣCONH―,

et 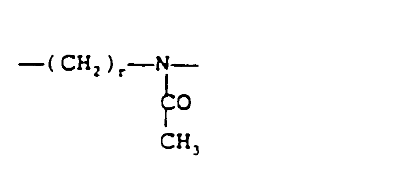 r étant un nombre entier de 1 à 5.

R₃ est avantageusement choisi parmi: 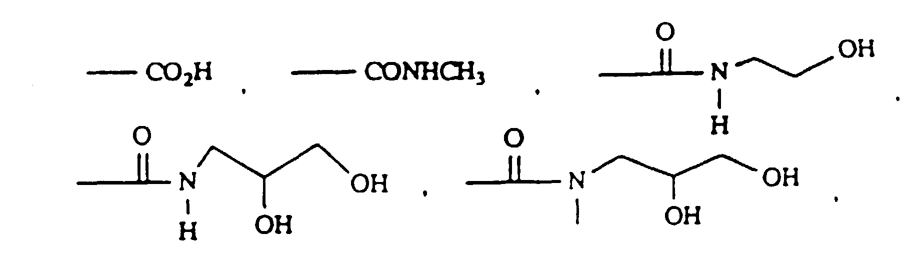 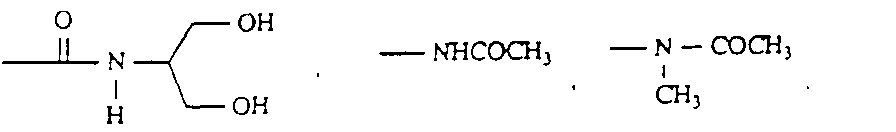 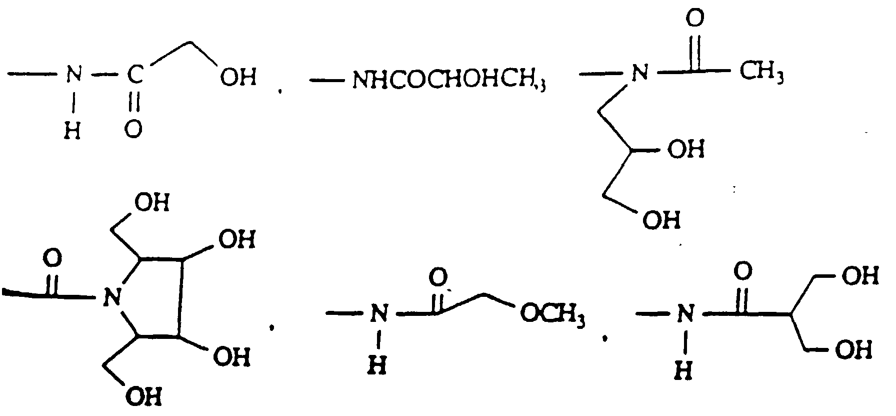 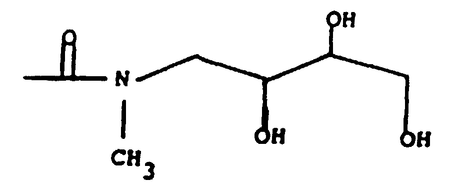 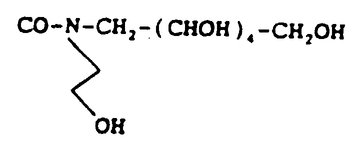 -CO-N-(CH₂-(CHOH)₄-CH₂OH)₂ -CO-NH-CH₂-C (CH₂-O-CH₂(CHOH)ₙ-CH₂OH)₃, n = 1 à 4 -CO-NH-CH₂-C(CH₂-O-CH₂CHOHCH₂OH)₃

R₄ est avantageusement choisi parmi les groupes R₃ et un groupe de formule -Q-B₄, Q étant tel que défini ci-dessus et B₄ représentant un groupe de formule: 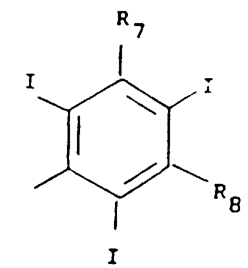 R₇ et R₈ ayant les mêmes significations que R₃.

Un groupe de composés préférés selon l'invention est représenté par les composés répondant à la formule générale V : 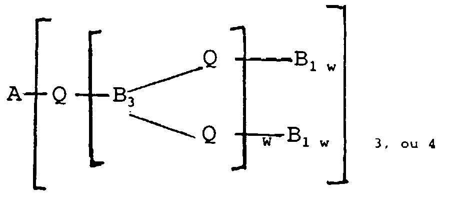 w = Σ 2ⁿ et w' = 2ⁿ (n représentant un nombre entier de 0 à 4)
dans laquelle A et B₁ sont tels que définis précédemment, Q représente de préférence le groupe 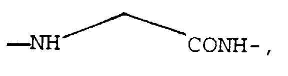 et
B₃ représente le groupe : 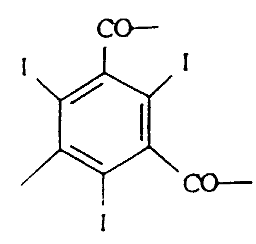 B₁ représente le groupe 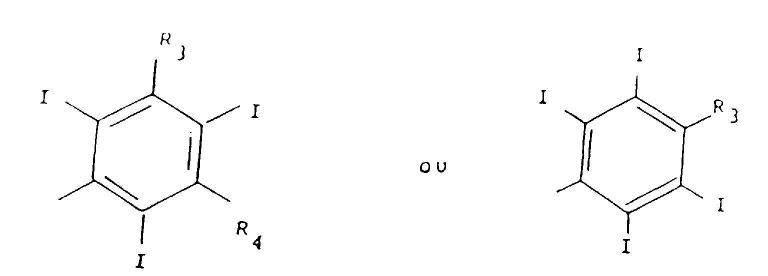 dans lesquelles
R₃ et R₄ sont choisis parmi CO⁻₂M⁺, 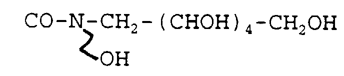 CO-N(CH₂(CHOH)₄-CH₂OH)₂

Ces composés possèdent une structure ramifiée, chaque groupe B₃ étant lié à trois groupes Q. Le degré de ramification (n) peut aller jusqu'à 4. Ainsi, la structure de ces composés obéit à une progression géométrique de raison 2 telle que définie ci-dessus.

Des groupes de composés préférés correspondant à ceux dont la formule générale suit et dans lesquels M⁺ possède l'une des valeurs indiquées plus haut : 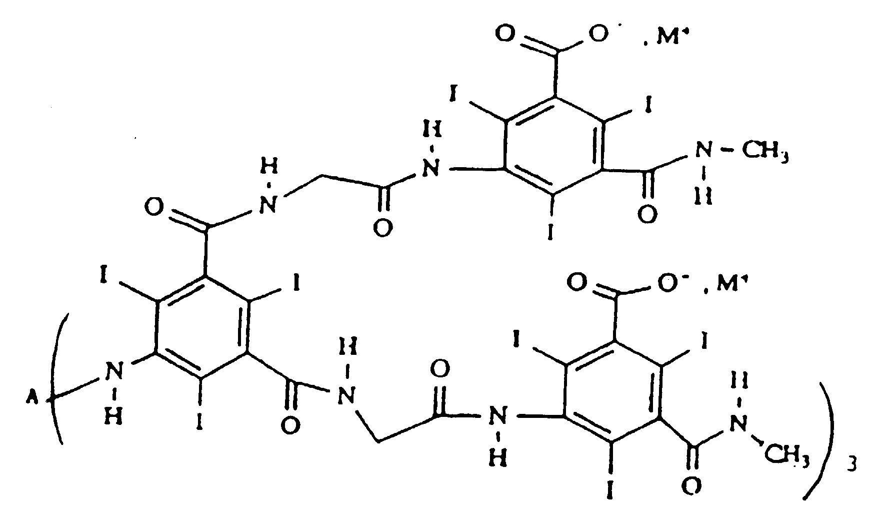 avec A =

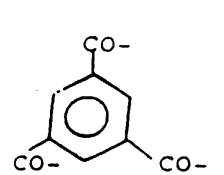

Les composés préférés sont ceux énumérés à la revendication 9.

Les composés polyiodés de l'invention peuvent être obtenus comme suit :
1.
   i) par activation d'un composé de formule A'(OH)ₘ, A' représentant A tel que défini ci-dessus et possédant m groupes Y représentant -CO, m étant tel que défini ci-dessus, les groupes OH de A' étant éventuellement protégés par un groupe protecteur, avec un réactif de chloruration pour obtenir un polychlorure d'acide de formule :

      A'―(―Cl)ₘ,

      suivie d'un
   ii) couplage du composé de formule :

      A'―(―Cl)ₘ

      avec m composés de formule X'H, X' représentant un groupe X tel que défini ci-dessus dans lequel Q, B₃ comporte au moins un groupe -NH-, -NR₅-, R₅ étant tel que défini ci-dessus, les groupes OH de X' étant éventuellement protégés par un groupe protecteur, pour obtenir un composé de formule:

      A'―(-X')ₘ
   iii) et déprotection des groupes hydroxy protégés de A' et X', de manière à obtenir un composé de formule A―(-X)ₘ,
   soit
2.
   i) activation directe d'un composé de formule A'(OH)ₘ, tel que défini ci-dessus,
   ii) couplage du composé ci-dessus avec m composés de formule X'H, X' étant tel que défini ci-dessus, de manière à obtenir un composé de formule :

      A'-(-X')ₘ

      telle que définie ci-dessus; et
   iii) déprotection des groupes OH protégés de A' et X'pour obtenir un composé de formule :

      A-(-X)ₘ,

      soit
3.
   i) réaction d'un composé de formule A'(H)ₘ dans lequel A' représente un groupe A tel que défini ci-dessus comportant m groupes Y représentant -NH ou -NR₅ et m étant tel que défini ci-dessus, les groupes OH de A' étant éventuellement protégés par un groupe protecteur, avec
      - m chlorures d'acide de formule X'-(-Cl)ₘ, X' représentant un groupe X tel que défini précédemment comportant au moins un groupe -CO, dont les groupes OH sont éventuellement protégés, de façon à obtenir un composé A'-(-X')ₘ, déprotection des groupes OH de A' et X' de façon à obtenir un composé de formule A-(-X)ₘ ;
   ii) réaction d'un composé de formule A'-(-H)ₘ dans laquelle A' représente un groupe A tel que défini ci-dessus comportant un groupe Y représentant -O- ou >NCOR₅, les groupes -OH de A' étant éventuellement protégés par un groupe protecteur, avec
      - m composés de formule X'-L, X' représentant un groupe X tel que défini précédemment dont les groupes OH sont protégés et L représente un groupe partant,
      de façon à obtenir un composé de formule A'-(-X')ₘ telle que définie ci-dessus, déprotection des groupes OH de A' et X', de façon à obtenir le composé de formule :

      A-(-X)ₘ.

La réaction de l'étape 1.i) a lieu avec un réactif de chloruration tel que SOCl₂, PCl₅, (CO)₂Cl₂ dans un solvant tel que le DMAC, le DMF, l'acétate d'éthyle, le dichlorométhane.

La réaction de l'étape 1.ii) a lieu de préférence dans un solvant tel que le DMF, le DMAC ou le dichlorométhane.

La réaction de l'étape 2.i) a lieu en présence d'un réactif de couplage, notamment la 1-éthoxycarbonyl-2-éthoxy-1,2-dihydroquinoline, le 1-(3-diméthylaminopropyl)-3-éthylcarbodiimide ou le 1,3-dicyclohexylcarbodiimide, dans un solvant tel que le DMAC, le DMF, le dichlorométhane, l'eau, éventuellement en présence d'un catalyseur tel que le l-hydroxy-lH-benzotriazole.

La réaction de l'étape 3i) est réalisée de préférence dans un solvant tel que le DMF, le DMAC, CH₂Cl₂, le dioxane.

Les chlorures d'acide de l'étape 3ii) de formule X"CO-Cl sont obtenus par exemple à partir des composés correspondants de formule X"COOH avec un réactif tel que SOCl₂, PCl₅, (CO)₂Cl₂ dans un solvant organique tel que le DMAC, le DMF ou CH₂Cl₂.

Le groupe partant L de l'étape 3.ii) est de préférence choisi parmi les groupes O-tosyle, O-mésyle, un atome d'halogène (Cl, Br, I).

Les produits de départ de formule A'(H)ₘ ou A"(COOH)ₘ sont obtenus comme indiqué dans les publications précédemment citées en référence au groupe A correspondant ou obtenus à partir de produits vendus dans le commerce (par exemple les cyclodextrines α, β et γ commercialisées par Sigma, France) ou préparés comme décrit ci-après.

La fixation des groupes Q sur les groupes B₁, et B₃ est réalisée de façon connue en soi par des réactions d'acylation, d'amidification ou d'alkylation des groupes B dans des solvants tels que le DMF, le DMAC, CH₂Cl₂, éventuellement en présence d'une base, notamment pour les réactions d'alkylation.

Les produits de départ triiodés correspondant aux groupes B₁ et B₃ sont obtenus notamment selon les méthodes décrites dans FR-A-2 272 640, FR-A- 2 632 304, FR-A-2 673 180 et FR-A-2 656 865 ou dans l'ouvrage "Radio Contrast Agents" (Sovak H. Ed. Sovak, Berlin, New York, Springer 1984).

Les composés de formule générale I, peuvent être obtenus à partir d'un composé de formule générale VI : 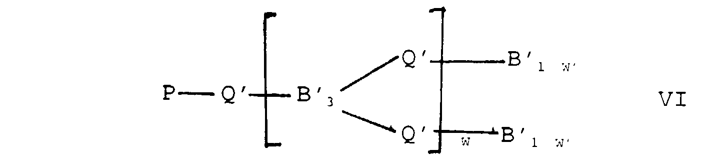 dans laquelle Q', B'₁, B'₃ représentent respectivement les groupes Q, B₁ et B₃ dont les groupes OH sont éventuellement protégés, w et w' sont tels que définis précédemment, et P est soit un atome d'hydrogène, soit un groupe protecteur usuel du groupe amino ou carboxy, soit un groupe partant (par exemple Cl, Br, I, OTs, OMs) que l'on couple après déprotection par élimination du groupe P sur le coeur de la molécule A par des réactions d'acylation, d'alkylation (amination réductrice) bien connues de l'homme du métier et on déprotège ensuite les fonctions OH éventuellement protégées pour obtenir le composé de formule générale I souhaité.

Par exemple, le couplage des composés de formule générale II est réalisé sur un composé de formule A'(OH)ₘ, par exemple l'acide trimésique, après activation des groupes carboxy par exemple à l'aide d'un carbodiimide, suivie d'une acylation éventuelle des fonctions amines libres du composé de formule générale VI.

Le composé de formule générale VI peut être préparé à partir des composés intermédiaires de formules VII et VIII suivantes :

P-Q'-B'₃(L)₂ VII

P-Q'-B'₁ VIII

dans lesquelles P représente H, ou un groupe protecteur des fonctions amines primaires ou secondaires bien connu de l'homme du métier, tel le groupe phtalimido ou un groupe protecteur de la fonction carboxy, et Q', B'₁, B'₃ représentent respectivement les groupes Q, B₁ et B₃ dont les fonctions hydroxy présentes sont éventuellement protégées, et L représente un groupe partant (par exemple Cl, Br, I, OTs ou OMs).

Ainsi, pour n = 0, le composé de formule VIII est déprotégé par élimination du groupe protecteur P (par exemple par hydrazynolyse des groupes phtalimido) et le composé ainsi déprotégé (par exemple l'amine) obtenu est mis à réagir avec un composé de formule VII (par exemple un dichlorure d'acide) dans les solvants usuels (DMAC, DMF, N-méthyl pyrrolidinone,...) en présence d'une base organique ou minérale pour obtenir un composé de formule

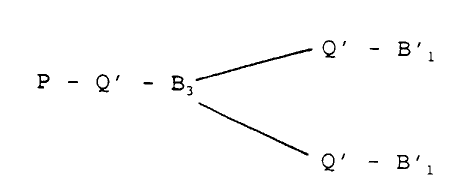

Pour n = 1, après élimination du groupe protecteur P (par exemple par hydrazynolyse des groupes phtalimido), le composé obtenu ci-dessus est mis à réagir avec un composé de formule VII dans les mêmes conditions pour obtenir un composé de formule IX

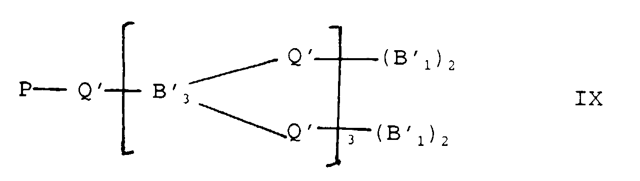

Pour n = 2,3,4, on fait réagir de la même manière successivement le composé obtenu à la fin de chaque étape dont le groupe protecteur a été éliminé sur un composé de formule VII pour obtenir un composé de formule générale VI.

L'invention a également pour objet des produits de contraste qui comprennent au moins un composé de formule I.

Ces produits de contraste sont utilisés chez l'homme et les animaux à des fins radiologiques.

La forme pharmaceutique préférée des produits de contraste selon l'invention est constituée par des solutions aqueuses ou des suspensions des composés, de préférence dans de l'eau bidistillée. Elle peut également se présenter sous la forme d'une poudre.

Les solutions aqueuses des composés de formule I peuvent également contenir certains additifs comme :
- du chlorure de sodium à des concentrations comprises entre 0,1 et 10 mM/l
- de l'EDTA disodique à des concentrations comprises entre 0,1 et 2 mM/l
- du citrate de sodium à des concentrations comprises entre 0,1 et 10 mM/l
- le chlorhydrate de tris(hydroxyméthyl)amino méthane,
- l'amine du tris(hydroxyméthyl)amino méthane,
- de l'héparine à des doses comprises entre 10 et 100 unités pour 100 ml de solution,
- le calciédétate de sodium.

Pour l'application en radiographie par les rayons X, le produit de contraste selon l'invention est administré à une dose utile variant d'environ 1 à 1000 ml de solution aqueuse, pour obtenir des concentrations variant d'environ 0,01 g d'iode/kg de poids corporel à environ 5 g d'iode/kg de poids corporel, de préférence d'environ 0,1 g d'iode/kg de poids corporel à 2 g d'iode/kg de poids corporel.

Ces compositions peuvent être administrées par toutes les voies classiquement utilisées pour les produits de contraste iodés. Ainsi, lorsqu'elles se présentent en solution, elles peuvent être administrées par voie entérale ou parentérale (voie orale, rectale, intraveineuse, intraartérielle, intra-articulaire, sous-arachnoïdienne, ainsi que par les voies bronchique, lymphatique et intra-utérine), de préférence les compositions sont administrées par voie intra-vasculaire.

Lorsqu'elles se présentent sous forme de suspension dans l'eau ou sous forme d'une poudre dans une formulation galénique physiologiquement acceptable, les compositions selon l'invention sont administrées de préférence par les voies entérale, orale, rectale ou bronchique.

Les compositions de l'invention peuvent en outre se présenter sous forme de liposomes, les composés de formule I étant encapsulés à l'intérieur de ces liposomes.

Dans le cas d'une injection, celle-ci peut se faire par bolus ou en perfusion.

On donnera ci-après un exemple de composition selon la présente invention.

| **Composition A** | |
|---|---|
| Composé de l'exemple 1 | 35,77 g |
| Eau pour préparation injectable Q.S.P. | 100 ml. |

| **Composition B** | |
|---|---|
| Composé de l'exemple 1 | 53,65 g |
| Eau pour préparation injectable Q.S.P. | 100 ml. |

L'invention a également pour objet une méthode de diagnostic par rayons X, notamment du compartiment vasculaire d'un sujet, caractérisée en ce qu'elle comprend l'administration par voie intraveineuse ou intra-artérielle d'un produit de contraste tel que défini ci-dessus, en une quantité suffisante pour rendre le compartiment vasculaire dudit sujet opaque aux rayons X et l'exposition dudit sujet à une dose définie de rayons X, la dose injectée variant d'environ 0,01 g d'iode/kg de poids corporel à environ 5 g d'iode/kg de poids corporel, de préférence d'environ 0,1 g d'iode/kg de poids corporel à environ 2 g d'iode/kg de poids corporel.

On décrira ci-après la préparation de composés entrant dans la synthèse des composés de l'invention ainsi que quelques exemples de composés selon l'invention.

### Préparation du composé AH₃ de formule :

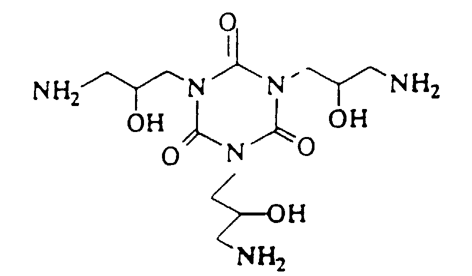

**1) Préparation de la N,N',N"-tri(époxy 2-3 propyl) 1,3,5-triazine 2,4,6(1H,3H,5H) trione** 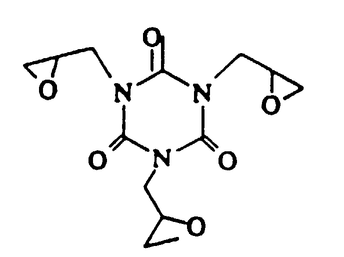
   Sur un mélange de 10 g (0,04 mole) de N,N',N"-triallyl 1,3,5-triazine 2,4,6-(1H,3H,5H) trione (disponible commercialement auprès de Aldrich - Strasbourg - France) et de 100 ml de dichlorométhane sont ajoutés 74 g (0,43 mole) d'acide métachloroperbenzoïque à 70 % en suspension dans 400 ml de dichlorométhane. L'agitation est maintenue pendant 6 jours à température ambiante.
   Après refroidissement et retour à température ambiante, la solution est filtrée; les peroxydes contenus dans le filtrat sont neutralisés avec 35 ml d'une solution de sulfite de sodium à 10 %. La phase organique décantée est lavée par 2 fois 100 ml de solution de bicarbonate de sodium 5 %. Après décantation, la phase organique est séchée sur sulfate de magnésium et évaporée.
   10 g de poudre blanche sont obtenus, soit un rendement de 84 %.
**2) Préparation de la N,N'N"-tri(2-hydroxyéthyliminodibenzyl) 1,3,5-triazine 2,4,6-(1H,3H,5H)-trione** 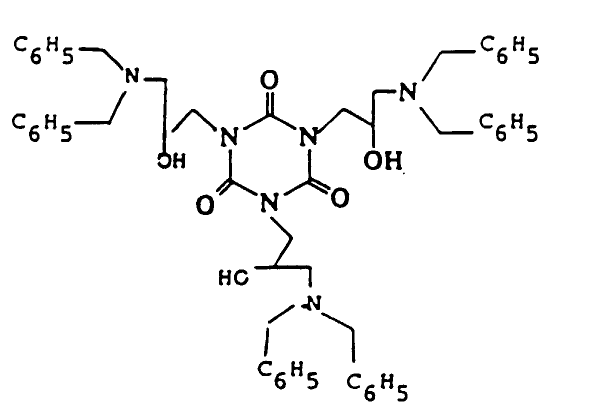
   4,97 g (0,025 mole) de dibenzylamine sont ajoutés à une solution de 2,5 g (0,008 mole) de N,N',N"-tri(2,3-époxypropyl)1,3,5-triazine 2,4,6 (1H,3H,5H)-trione dans 10 ml de dichlorométhane. L'agitation est maintenue pendant deux jours à température ambiante. Le milieu réactionnel est évaporé et le produit obtenu est purifié par chromatographie avec de l'acétate d'éthyle. Après évaporation de la phase organique, 3,6 g de poudre beige sont obtenus, soit un rendement de 50 %.
**3) Préparation de la N,N',N"-tri(amine-2-hydroxy-3-propyl) 1,3,5-triazine 2,4,6-(1H,3H,5H)-trione** 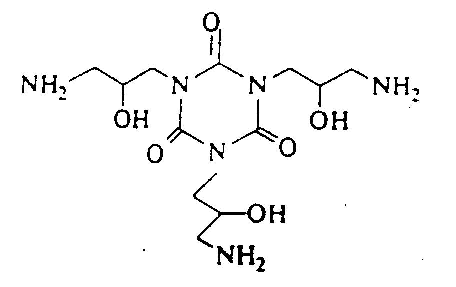
   2 g (0,002 mole) de N,N',N"-tri(2-hydroxyéthyliminodibenzyl) 1,3,5-triazine 2,4,6 (1H,3H,5H)-trione en solution dans 200 ml de méthanol sont hydrogénés sous 8 bars à 50° C en présence de charbon palladié à 50 % hydraté pendant 3 heures.

Après filtration et évaporation, 500 mg d'une poudre blanche sont obtenus, soit un rendement de 70 %.

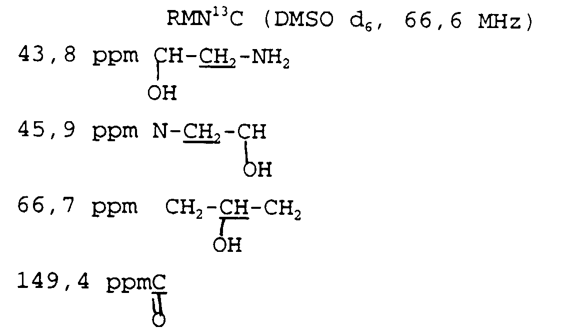

### Préparation du composé A(OH)₃ de formule :

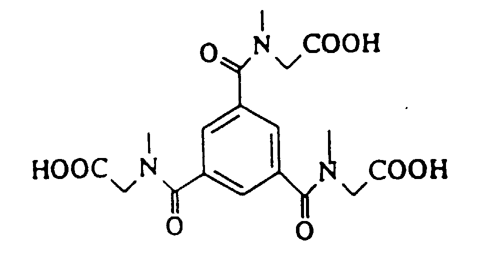

**1) Estérification de la sarcosine**
   L'estérification de la sarcosine est réalisée selon la méthode décrite dans Agric. Biol. Chem., 50 (3) 615, 1986.
**2) Chloruration de l'acide trimésique**
   73 g (0,35 mole) d'acide trimésique (disponible commercialement chez la société SIGMA, France) sont mis en suspension dans 300 ml de chlorure de thionyle. Après un reflux de 24 h, élimination du chlorure de thionyle et distillation sous vide, le trichlorure d'acide est obtenu sous forme d'un solide blanc.
   (88,1 g, Rendement : 95,5 %, P.F. : 35° C).
   2,62 g (0,019 mole) d'ester de la sarcosine sous forme de chlorhydrate obtenu ci-dessus, 6,55 ml (0,047 mole) de triéthylamine, puis 1 g (0,0038 mole) de trichlorure d'acide trimésique sont ajoutés lentement dans le dichlorométhane. Après 6 h de reflux, refroidissement, filtration, 1,55 g de produit sous forme de triester sont obtenus, soit un rendement de 89 %.
   Rf (CH₂Cl₂:MeOH 9/1) 0,47
   RMN¹H DMSO d₆ 200 MHz
   2,99 (s, 9H, NH-CH₃); 3,68 (s, 9H,CO₂CH₃); 4,16 (s,6H,CH₂); 7,43 (s,3H,CH).
**3) Saponification du triester**
   0,61 g (0,0013 mole) du triester préparé à l'étape précédente sont mis en solution dans 8 ml de méthanol. 0,52 g (0,013 mole) de soude sont ajoutés en solution dans 2 ml d'eau. Après 12 h à température ambiante, acidification, filtration et évaporation, 0,55 g de triacide sont obtenus.
   RMN¹H DMSO d₆ 200 MHz
   2,95 (s,9H,CH-CH₃); 4,1 (s,6H,CH₂); 7,4 (s,3H,CH); 7,77 (s,3H, COOH).

### Préparation du composé AH₄ de formule :

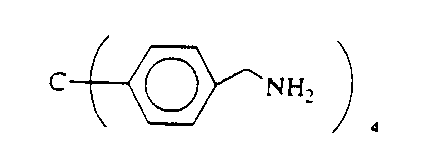

La préparation se fait à partir du composé: 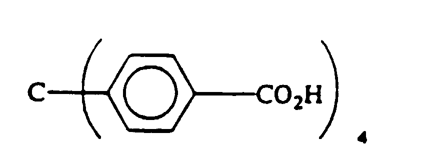 préparé selon la méthode décrite dans "Angew. Chem. Int. Ed. Engl. 25, 1986, 1097.

Le passage -phényl-CO₂H → phényl NH₂ est réalisé en suivant la méthode décrite dans JACS, 101, P. 2728, 1979.

Pour illustrer la méthode de synthèse on décrit tout d'abord la préparation du composé de formule X :

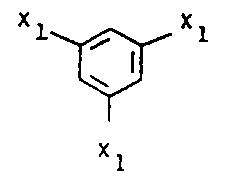

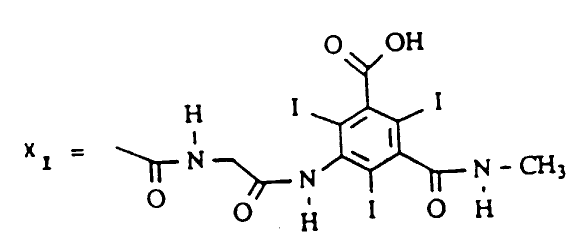

4 g (0,019 mole) d'acide trimésique, 37,11 g (0,059 mole) d'acide 3-N-méthylcarbamoyl 5-aminoacétamido-2,4,6-triiodobenzoïque préparé selon la méthode décrite dans le brevet US 4 014 986, 10,49 g (0,0685 mole) d'hydroxybenzotriazole, 10 ml de triéthylamine et 13,14 g (0,0685 mole) de chlorhydrate de 1-(3-diméthyl aminopropyl)-3-éthylcarbodiimide (EDCI) sont mis en soluion dans 1 1 de DMF. Après agitation à température ambiante pendant 12h, puis évaporation du solvant, le résidu est trituré dans CH₂Cl₂. Le solide obtenu est purifié par dissolution dans l'ammoniaque puis mis à précipiter en milieu acide.

32,7 g de produit sous forme de poudre blanche sont obtenus, soit un rendement de 84 %.
CCM SiO₂ (AcOEt/isopropanol/NH₃ : 35/35/40)
Rf : 0,16
RMN¹H DMSO d₆, 200 MHz.
2,7 (s, 9H, CH₃); 3,3 (m, 3H, CO₂H); 4,2 (s, 6H, CH₂); 8,45 (dq, 3H, CO-NH-CH₃); 8,6 (s, 3H, CH); 9,05 (t, 3H, CO-NH-CH₂); 10,15 (d, 3H, Cₐᵣ-NH-CO)
RMN¹³C DMSO d₆ 66,6 MHz
26 (3CH₃) ; 43 (3CH₂); 88,8, 97,5, 99,2 (9 Cₐᵣ-I); 129 (3Cₐᵣ-H); 134,4 (3 Cₐᵣ CO); 143, 149, 150,5 (9 Cₐᵣ-1); 165,6, 167,5, 169,7 (12CO),

### EXEMPLE 1

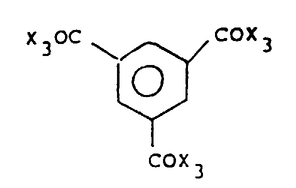

avec 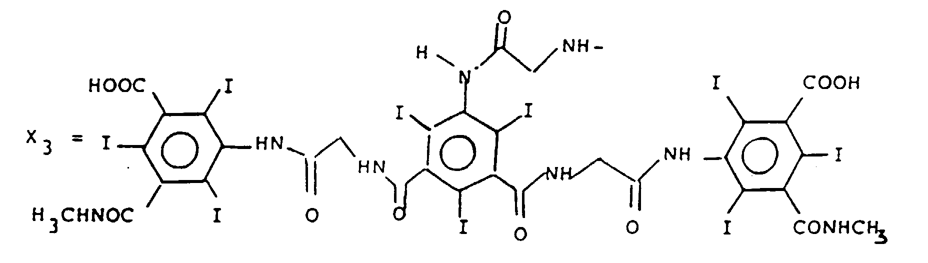
**1) Préparation du composé de formule :** 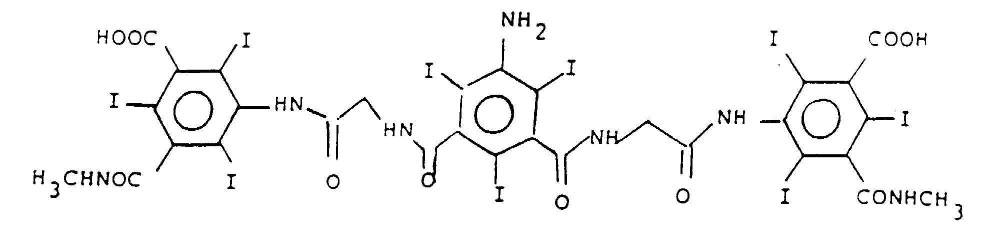 **N-N'bis-[N-(2,4,6-triiodo-3-carboxy 5-N-méthyl) phényl carbamoyl méthyl] 5-amino-2,4,6-triiodo-isophtalamide**
   26,4 g (0,042 mole) d'acide 2,4,6-triiodo-5-amino-N-méthyl isophtalamique sont ajoutés rapidement à une solution de 10 g (0,0168mole) de chlorure de 5-amino-2,4,6-triiodoisophtaloyle dans 30 cm³ de diméthylacétamide sous agitation. Le mélange est ensuite chauffé à 50 C pendant 3 h puis précipité dans HCl (10N). Après agitation pendant 24 h, le précipité est filtré, lavé à l'eau et au dichlorométhane puis séché.
   On obtient 20 g (67%) de produit.
   CCM (SiO₂) toluène/méthyléthylcétone/HCOOH : 60/25/20. Rf = 0,13. 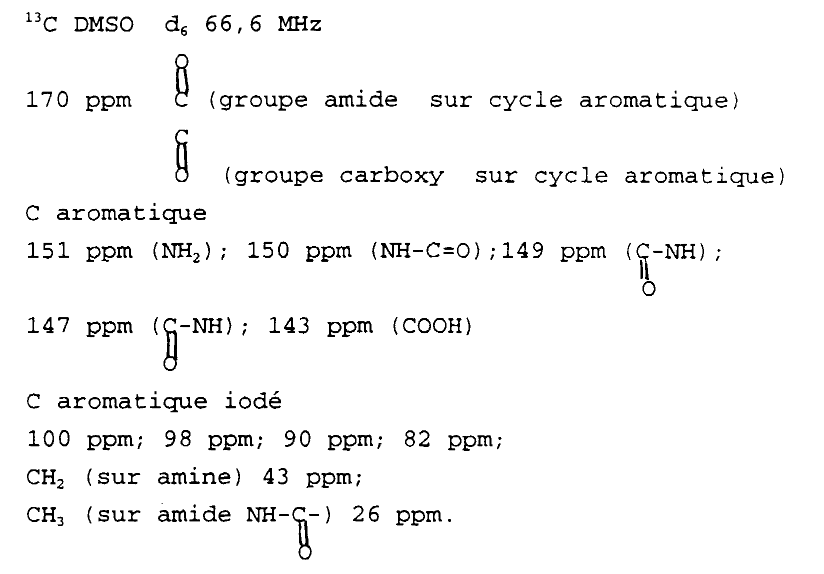
**2) Préparation du composé de formule :** 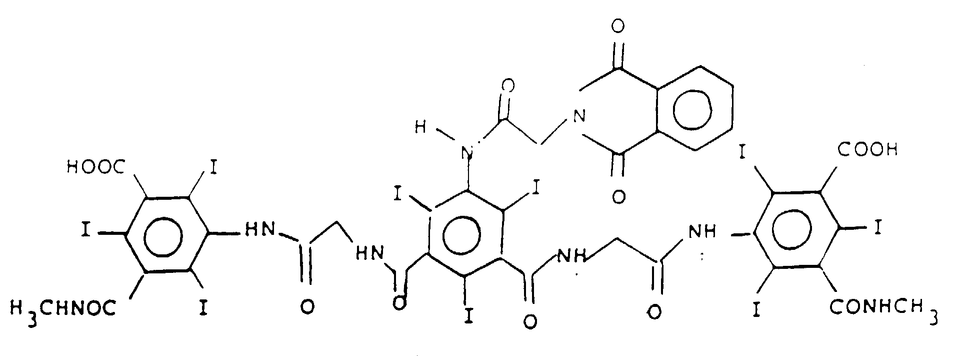 **N,N'bis [N-(2,4,6-triiodo-3-carboxy 5-N-méthyl) phényl carbamoylméthyl]5-N(acétylphtalimido)amino-2,4,6-triiodoisophtalamide**
   1,2 g (5,37.10⁻³ mole) de chlorure d'acide phtalylglycyle est ajouté à une solution de 1 g (5,48. 10⁻⁴ mole) du produit obtenu à l'étape précédente dans 2 cm³ de diméthylacétamide sous agitation. Le milieu réactionnel est agité à température ambiante pendant 24 h puis est précipité dans de l'eau chaude. Le précipité est filtré, lavé à l'eau chaude puis au dichlorométhane et séché.
   CCM (SiO₂) acétate d'éthylelisopropanol/NH₃: 35/35/40. Rf = 0,27.
   Le produit obtenu est déprotégé de façon classique par traitement à l'hydrazine. Après couplage à l'acide trimésique selon la méthode décrite ci-dessus pour le composé X, le produit du titre est obtenu.

### EXEMPLE 2

**1) Préparation du composé de formule :** 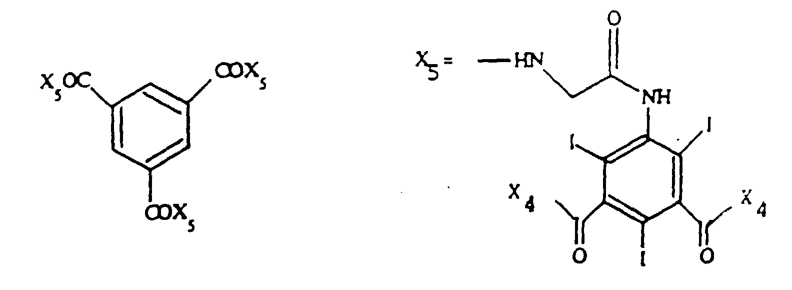 avec X₄ représente : 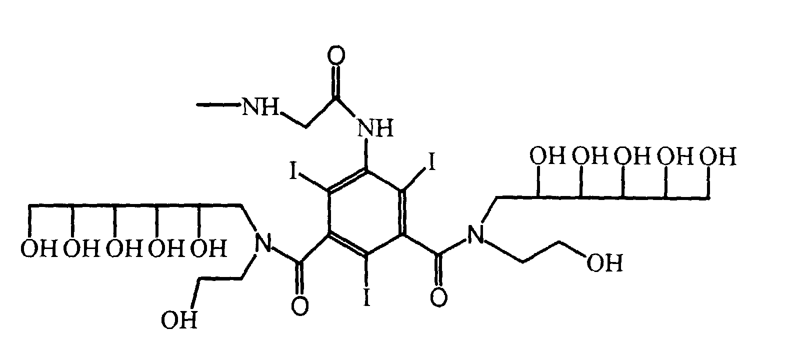
**1) Préparation du composé de formule :** 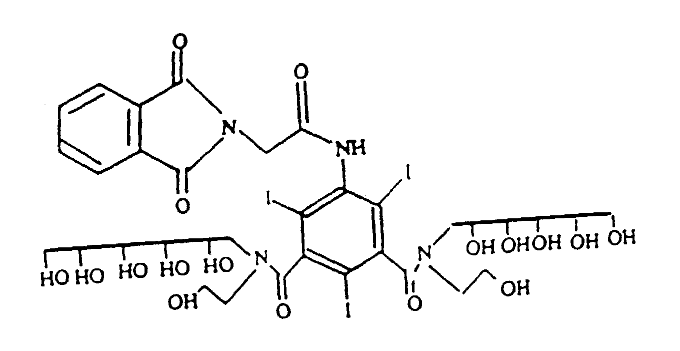
   300 g (0,38 mole) du chlorure de 5-N(acétylphtalimido) amino 2,4,6-triiodoisophtaloyle sont ajoutés à une solution de 258,9 g (1,149 mole) de 1-déoxy-l-(2-hydroxyéthylamino)-D-glucitol et de tributylamine (273,9 ml; 1,149 mole) dans 2 litres de diméthylacétamide. Le mélange est agité pendant une nuit à température ambiante. Après évaporation du solvant et précipitation dans le dichlorométhane, le produit est mis en solution dans l'eau et purifié par passage sur une résine échangeuse d'ions (Amberlite IRN 77). Après évaporation, on obtient 350 g (Rdt 80 %) de produit.
   ¹³C DMSO d₆ 66,6 MHz :
   170, 167, 166, 165 ppm (C=O)
   149, 143 ppm (C aromatique)
   135, 132, 123 ppm (C aromatique phtalimido)
   99, 91 ppm (C aromatique iodé)
   73, 71, 69 ppm (CH amine-alcool)
   63, 59, 58,5, 57 ppm (CH₂ amine-alcool)
   52,5, 52, 49, 48,5 ppm (CH₂ en α N de l'amine-alcool)
   41 ppm (CH₂ en α du phtalimido).
**2) Préparation du composé de formule : HX**_{**4**}
   275 g (0,22 mole) du composé préparé à l'étape 1 sont mis en solution dans 1,4 litre d'eau en présence de 33,2 ml (0,66 mole) d'hydrate d'hydrazine. Le mélange est chauffé à 80° C pendant 2 heures puis, après retour à température ambiante, acidifié avec 53 ml d'acide chlorhydrique (10N). L'isoluble est filtré et la solution est purifiée par passage sur résines échangeuses d'ions (Amberlite IRA 67 et IRC 50). Après évaporation, on obtient 188,7 g de produit (Rdt = 97 %).
   ¹³C DMSO d₆ 66,6 MHz :
   173, 171, 170 ppm (C = O)
   149, 143 ppm (C aromatique)
   99, 91 ppm (C aromatique iodé)
   73, 71, 69 ppm (CH amine alcool)
   63, 59, 58,5, 57 ppm (CH₂ amine alcool)
   52,5, 52, 49, 48,5 ppm (CH₂ en α N amine alcool)
   45 ppm (CO - CH₂ - NH₂).
**3) Préparation du composé de formule :** 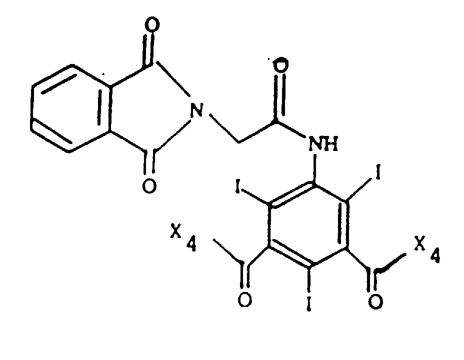
   150 g (0,146 mole) du composé obtenu à l'étape 2 sont ajoutés à une solution de 400 ml de diméthylacétamide contenant 35 ml de tributylamine et 47,5 g (0,06 mole) de chlorure de 5-N(acétylphtalimido)amino 2,4,6-triiodo- isophtaloyle. Le milieu réactionnel est chauffé à 50° C pendant 2 heures puis concentré à sec. Le produit est ensuite trituré dans le chlorure de méthylène puis purifié par chromatographie sur silice silanisée (SiRP2, élution à l'eau) . On obtient ainsi 110 g (Rdt = 80 %) du produit attendu.
   ¹³C DMSO d₆ 66,6 MHz
   170, 167, 166, 165 ppm (C = O)
   149, 148,5, 143,5, 143 ppm (C aromatique)
   135, 132, 123 ppm (C aromatique phtalimido)
   99,5, 99, 91, 90,5 ppm (C aromatique iodé)
   73, 71, 69 ppm (CH amine alcool)
   63, 59, 58,5, 57 ppm (CH₂ amine alcool)
   52,5, 52, 49, 48,5 ppm (CH₂ en α N amine alcool)
   44 ppm (CO-NH-CH₂-CONH)
   41 ppm (CH₂ en α phtalimido).
**4) Préparation du composé de formule : X**_{**5**}**H**
   110 g (0,04 mole) du composé obtenu à l'étape 3) sont mis en solution dans 370 ml d'eau en présence de 6 ml (0,12 mole) d'hydrate d'hydrazine. Le mélange est chauffé à 80° C pendant 2 heures puis, après retour à température ambiante, acidifié avec 16 ml d'acide chlorhydrique (10 N). L'insoluble est filtré et la solution est purifiée par passage sur résines échangeuses d'ions (Amberlite IRA67 et IRL50). Après évaporation on obtient 99 g de produit (Rdt = 94 %).
   ¹³C DMSO d₆ 66,6 MHz
   170, 168, 165 ppm (C = O)
   149, 148,5, 143,5, 143 ppm (C aromatique)
   99,5, 99, 91, 90,5 ppm (C aromatique iodé)
   73, 71, 69 ppm (CH amine alcool)
   63, 59, 58,5, 57 ppm (CH₂ amine alcool)
   52,5, 52, 49, 48,5 ppm (CH₂ en *a* N amine alcool)
   45 ppm (NH-CO-CH₂-NH₂)
   43 ppm (CONH-CH₂-CONH)
**5) Préparation du composé du titre**
   Par réaction entre le composé obtenu à l'étape 4) et l'acide trimésique dans les conditions habituelles de couplage, on obtient avec un rendement de 80% le composé du titre.
   ¹³C DMSO d₆ 66,6 MHz
   170, 167, 166, 165 ppm (C = O)
   149, 148,5, 143,5, 143 ppm (C aromatique)
   135, 127 ppm (C aromatique trimésique)
   100, 99,5, 90,5, 90 ppm (C aromatique iodé)
   73, 71, 69 ppm (CH amine alcool)
   63, 59, 58,5, 57 ppm (CH₂ amine alcool)
   52,5, 52, 49, 48,5 ppm (CH₂ en α N amine alcool)
   43 ppm (CONH-CH₂-CONH).

### EXEMPLE 3

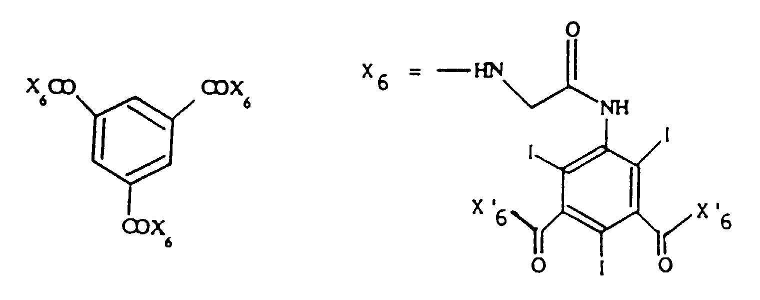

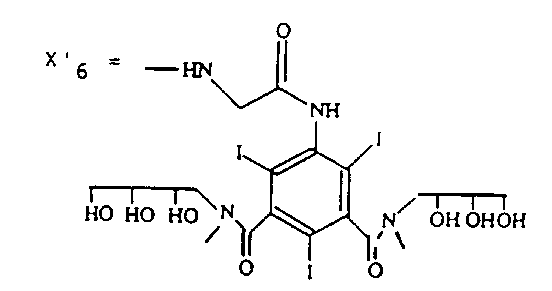

**1) Préparation du composé de formule :** 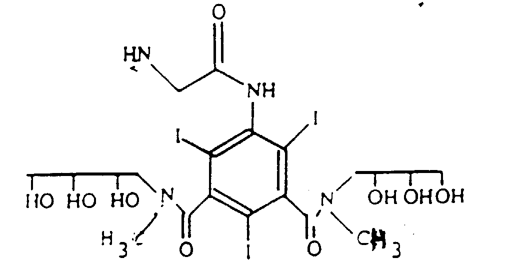
   150 g (0,19 mole) du chlorure de 5-N(acétylphtalimido)- amino 2, 4, 6-triiodoisophtaloyle sont mis en réaction avec 78 g (0,578 mole) de N-méthylaminobutanetriol suivant le mode opératoire décrit à l'étape 1) de l'exemple 2. On obtient ainsi 186,2 g (Rdt = 99 %) du produit de diamidification qui est hydrazinolysé dans les conditions de l'exemple 7.2 pour obtenir avec un rendement de 74 % le produit attendu.
   ¹³C DMSO d₆ 66,6 MHz
   171, 170, 165 ppm (C = O)
   150, 144 ppm (C aromatique)
   99, 90 ppm (C aromatique iodé)
   72, 66 ppm (CH amine alcool)
   63 ppm (CH₂ amine alcool)
   55, 51 ppm (CH₂ en α N amine alcool)
   45 ppm (NH-CO-CH₂-NH₂)
   37, 34 ppm (CH₃-N).
**2) Préparation du composé de formule : X**_{**6**}**H**
   80 g (0,094 mole) du composé obtenu à l'étape 1) sont mis en réaction avec 30,7 g (0,039 mole) de chlorure de 5-N (acétylphtalimido)amino 2,4,6-triiodoisophtalazoyle en suivant le mode opératoire de l'exemple 9.1. On obtient ainsi 72,4 g (Rdt = 77 %) du produit de diamidification qui est hydrazinolysé dans les conditions de l'exemple 9.2 pour obtenir avec un rendement de 91 % le produit attendu.
   ¹³C DMSO d₆ 66,6 MHz
   171, 170, 165 ppm (C = O)
   150, 149,5, 144,5, 144 ppm (C aromatique)
   100, 99,5, 90,5, 90 ppm (C aromatique iodé)
   72, 66 ppm (CH amine alcool)
   63 ppm (CH₂ amine alcool)
   55, 51 ppm (CH₂ en α N amine alcool)
   45 ppm (NHCO-CH₂-NH₂)
   44 ppm (CONH-CH₂-CONH)
   37,34 ppm (CH₃-N).
**3) Préparation du composé du titre**
   Par réaction entre le composé obtenu à l'étape 2) et l'acide trimésique dans les conditions habituelles de couplage décrites à l'exemple 2, on obtient avec un rendement de 90 % le composé du titre.
   ¹³C DMSO d₆ 66,6 MHz :
   170, 167, 166, 165 ppm (C = O)
   150, 149,5, 144,5, 144 ppm (C aromatique)
   135, 127 ppm (C aromatique trimésique)
   100, 99,5, 90,5, 90 ppm (C aromatique iodé)
   72, 66 ppm (CH amine alcool)
   63 ppm (CH₂ amine alcool)
   55, 51 ppm (CH₂ en α N amine alcool)
   44 ppm (-CO-NH-CH₂-CONH)
   37, 34 ppm (CH₃-N).

### EXEMPLE 4 :

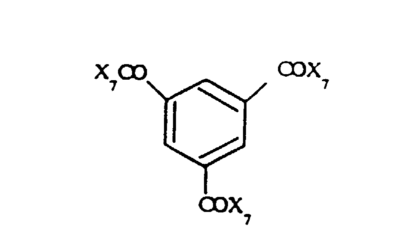

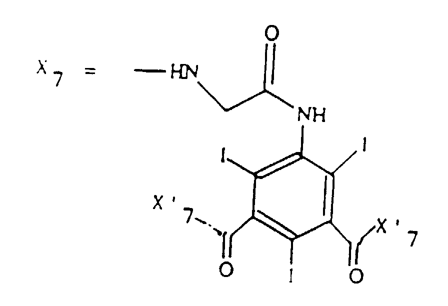

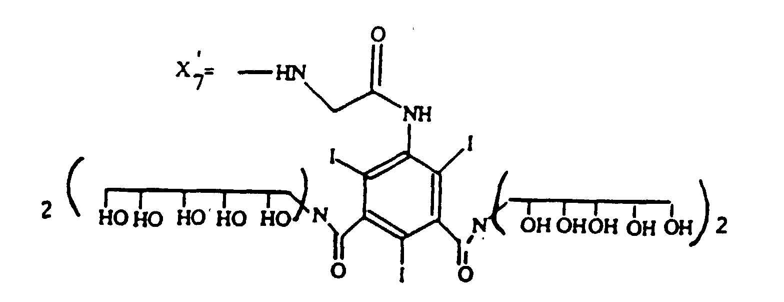

**1) Préparation du composé de formule : X**_{**7**}**H**
   208 g (0,24 mole) du chlorure de 5-N(acétylphtalimido) amino-2,4,6-triiodoisophtaloyle sont ajoutés à une solution de 416 g (1,2 mole) de bis-(1-déoxy-D-sorbit-l-yl)-amine et de 100 ml (0,72 mole) de triéthylamine dans 2 litres de 1-méthyl-2-pyrrolidinone (NMP). Le mélange est chauffé à 70°C pendant 24 heures. L'insoluble est alors filtré et le solvant est éliminé sous vide. Le produit est alors purifié comme à l'étape 1) de l'exemple 2) puis hydrozinolysé dans les conditions habituelles (étape 2) de l'exemple 2). Il est alors mis en réaction avec 0,42 équivalent de chlorure de 5-N(acétylphtalimido)amino-2,4,6-triiodoisophtaloyle dans les conditions de l'exemple 9.1 puis hydrazinolysé dans les conditions habituelles (exemple 9.2). Le produit est obtenu avec un rendement global de 51 %.
   ¹³C DMSO d₆ 66,6 MHz
   170, 167, 166, 165 ppm (C = O)
   149, 148,5, 143, 142,5 ppm (C aromatique)
   99, 98,5, 91, 90,5 ppm (C aromatique iodé)
   73, 71, 69 ppm (CH amine alcool)
   63 ppm (CH₂ amine alcool)
   54,5, 54, 49,5, 49 ppm (CH₂ en α N amine alcool)
   44 ppm (NHCO-CH₂-NH₂)
   43 ppm (CONH-CH₂-CONH).
**2) Préparation du composé du titre**
   Par réaction entre le composé de l'exemple 11.1 et l'acide trimésique dans les conditions habituelles de couplage décrites à l'exemple 2), on obtient avec un rendement de 80 % le produit du titre.
   ¹³C DMSO d₆ 66,6 MHz
   170, 167, 166, 165 ppm (C = O)
   149, 148,5, 143, 142,5 ppm (C aromatique)
   135, 127 ppm (C aromatique trimésique)
   99, 98,5, 91, 90,5 ppm (C aromatique iodé)
   73, 71, 69 ppm (CH amine alcool)
   63 ppm (CH₂ amine alcool)
   54,5, 54, 49,5, 49 ppm (CH₂ en α N amine alcool)
   44 ppm (CONH-CH₂-CONH).

### EXEMPLE 5 :

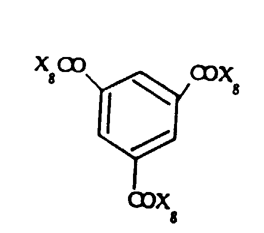

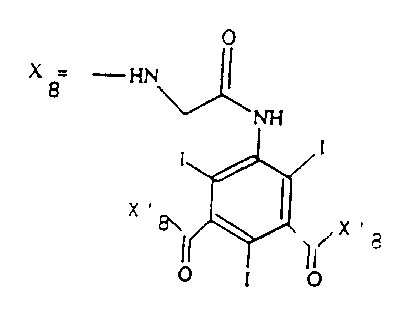

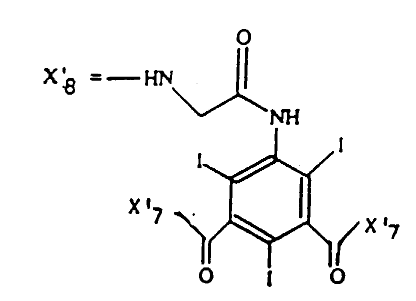

X'₇ étant tel que défini à l'exemple 4.

Le produit obtenu à l'étape 1) de l'exemple 4 est mis en réaction avec 0,42 équivalent de chlorure de 5-N(acétylphtalimido) amino-2,4,6-triioisophtaloyle dans les conditions décrites à l'étape 3) de l'exemple 2) puis hydrazinolysé de façon habituelle (étape 4 de l'exemple 2). Puis par couplage avec l'acide trimésique dans les conditions habituelles décrites à l'exemple 2), pour obtenir le produit du titre.

### EXEMPLE 6

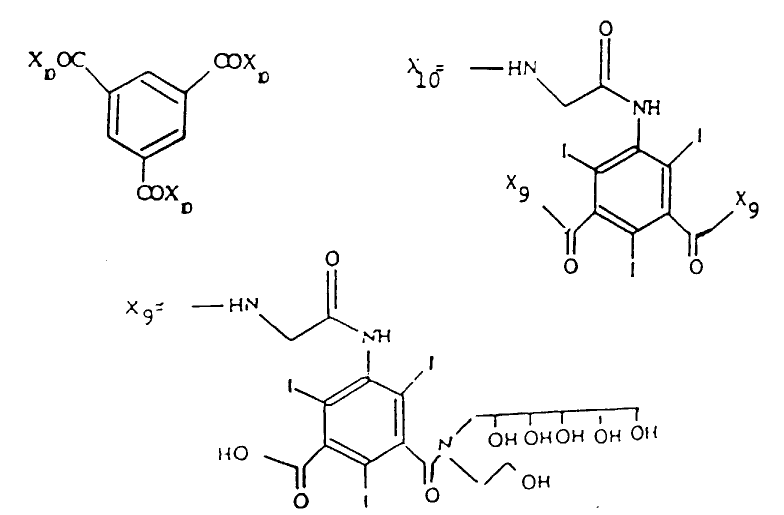

**1) Préparation du composé de formule :** 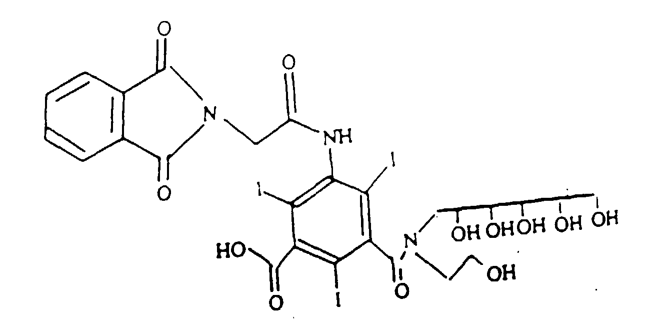
   119,5 g (0,13 mole) du chlorure de 5-N(acétylphtalimido) amino-2,4,6-triiodoisophtaloyle sont ajoutés à une solution de 41,53g (0,14 mole) de 1-déoxy-1-(2-hydroxyéthylamino)-D-glucitol et de triéthylamine (19,5 ml, 0,14 mole) dans 700 ml de diméthylacétamide. Le mélange est agité à température ambiante pendant 24 heures. 300 ml d'eau sont ajoutés et l'agitation est maintenue pendant 48 heures en chauffant à 45° C. Le solvant est alors évaporé sous vide et le produit est purifié sur résine échangeuse d'ions (Amberlite IRN 77) et sur silice silanisée (SiRP2, élution à l'eau). On obtient 26 g (Rdt = 38 % de produit).
   ¹³C DMSO d₆ 66,6 MHz
   170, 169,5, 167, 165 ppm (C = O)
   150, 147, 142 ppm (C aromatique)
   135, 132, 124 ppm (C phtalimido)
   100, 98, 90 ppm (C aromatique iodé)
   73, 71, 69 ppm (CH amine alcool)
   63, 59, 58 ppm (CH₂ amine alcool)
   52,5, 52, 49, 48,5 ppm (CH₂ en α N amine alcool)
   41 ppm (CH₂ en α phtalimido).
**2) Préparation du composé du titre :**
   Le produit obtenu à l'étape 1) est mis en réaction avec 0,42 équivalent de chlorure de 5-N(acétylphtalimido)amino-2,4,6-triiodoisophtaloyle dans les conditions de l'étape 3) de l'exemple 2) puis hydrazinolysé de façon habituelle (étape 4) de l'exemple 2)). Puis par couplage avec l'acide trimésique dans les conditions de l'exemple 2 le produit du titre est obtenu avec un rendement global de 12 %.
   ¹³C DMSO d₆ 66,6 MHz
   170, 167, 166, 165 ppm (C = O)
   150, 149,5, 147, 146,5, 142, 142,5 ppm (C aromatique)
   135, 128 ppm (C aromatique trimésique)
   100, 99,5, 98, 97,5, 90,5, 90 ppm (C aromatique iodé)
   73, 71, 69 ppm (CH amine alcool)
   63, 59, 58 ppm (CH₂ amine alcool)
   52,5, 52, 49, 48,5 ppm (CH₂ en α N amine alcool)
   44 ppm (CONH-CH₂-CONH).

## Revendications

1. Composés polyiodés ayant un poids moléculaire supérieur à 2000 et inférieur à 50000, de formule générale
A (̵X)ₘ
dans laquelle:
- A représente le reste d'une molécule polyfonctionnelle comportant un atome de carbone central tri- ou tétra-substitué, un atome central d'azote ou de phosphore tri-substitué ou un groupe P=O tri-substitué et/ou au moins un cycle carboné aromatique ou non, comportant éventuellement un ou plusieurs atomes d'iode, ou au moins un hétérocycle aromatique ou non aromatique comportant de 1 à 4 hétéroatomes choisis parmi O, S, N et P, sur laquelle sont fixés les m groupes X par l'intermédiaire de m liaisons ou de m groupes choisis parmi -CO-, 〉N-R₅, -O-, 〉NCOR₅, avec R₅ représentant H, un groupe alkyle linéaire ou ramifié en C₁-C₁₀, hydroxy- ou polyhydroxyalkyle linéaire ou ramifié en C₁-C₁₀, alkoxy (en C₁-C₅) alkyle linéaire ou ramifié en C₁-C₁₀, hydroxy- ou polyhydroxy- alkoxy (en C₁-C₅) alkyle linéaire ou ramifié en C₁-C₁₀;
- Les groupes X, identiques ou différents, représentent; 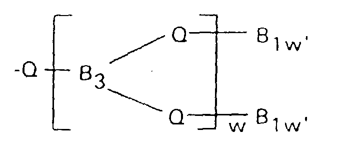 avec w = Σⁿₒ2ⁿ et w' = 2ⁿ, n représentant un entier de 0 à 4,
et les groupes Q, identiques ou différents entre eux, représentent une liaison simple ou un groupe choisi parmi: 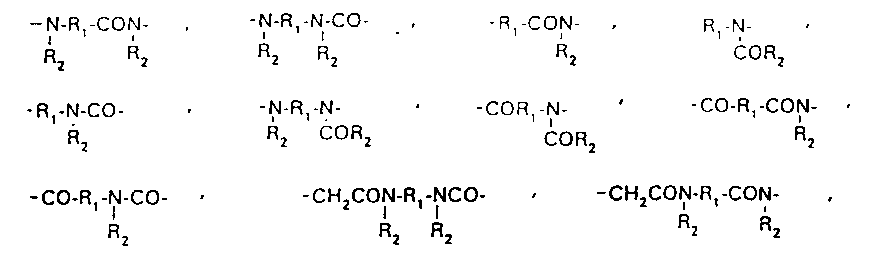 dans lesquels R₁ est choisi parmi une liaison simple et les groupes alkylène à chaîne linéaire ou ramifiée en C₁-C₁₀, hydroxy- ou polyhydroxy- alkylène à chaîne linéaire ou ramifiée en C₁-C₁₀, alkoxy (en C₁-C₅) alkylène à chaîne linéaire ou ramifiée en C₁-C₁₀, hydroxy- ou polyhydroxy- alkoxy (en C₁-C₅) alkylène à chaîne linéaire ou ramifiée, la chaîne alkylène étant éventuellement interrompue par un ou des atomes d'oxygène, les groupes R₂, identiques ou différents, sont choisis parmi H, les groupes alkyle à chaîne linéaire ou ramifiée en C₁-C₁₀, hydroxy- ou polyhydroxy- alkyle à chaîne linéaire ou ramifiée en C₁-C₁₀, alkoxy (en C₁-C₅) alkyle à chaîne linéaire ou ramifiée en C₁-C₁₀, hydroxy- ou poly-hydroxy- alkoxy (en C₁-C₅) alkyle à chaîne linéaire ou ramifiée;
et les groupes B₁, identiques ou différents entre eux, sont représentés par les formules: 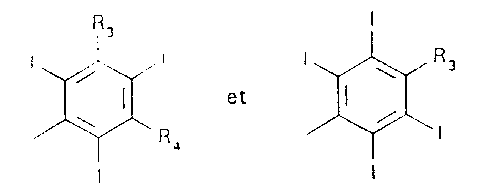 dans lesquelles R₃ est choisi parmi les groupes -COO⁻M⁺, avec M⁺ représentant H⁺ ou le cation physiologiquement acceptable d'une base organique ou minéral 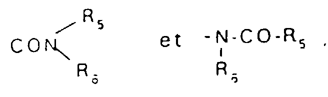 dans lesquels R₅ et R₆, identiques ou différents entre eux, sont définis comme précédemment pour R₅ ou R₅ et R₆ forment ensemble un groupe alkylène en C₄-C₈, hydroxyalkylène en C₄-C₈ ou polyhydroxyalkylène en C₄-C₈, à chaîne linéaire ou ramifiée éventuellement interrompue par un ou plusieurs atomes choisis parmi S, O, P et N, de sorte que R₅ et R₆ forment avec l'atome d'azote auquel ils sont liés un hétérocycle azoté de 5 à 12 chaînons éventuellement substitué par un ou plusieurs groupes hydroxy, hydroxy- ou polyhydroxyalkyle en C₁-C₄ à chaîne linéaire ou ramifiée et renfermant éventuellement un ou plusieurs hétéroatomes supplémentaires choisis parmi S, O, P et N, R₄ représente un groupe choisi parmi R₃ et Q-B₄, Q étant tel que défini précédemment et B₄ représentant un groupe : 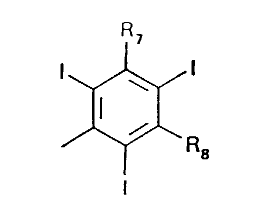 dans lequel R₇ et R₈, identiques ou différents entre eux, représentent R₃,
et les groupes B₃, identiques ou différents entre eux, sont choisis parmi: 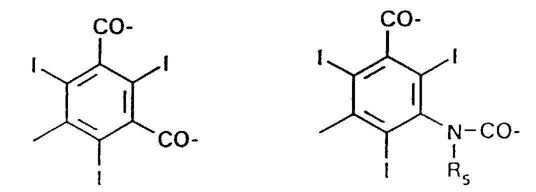 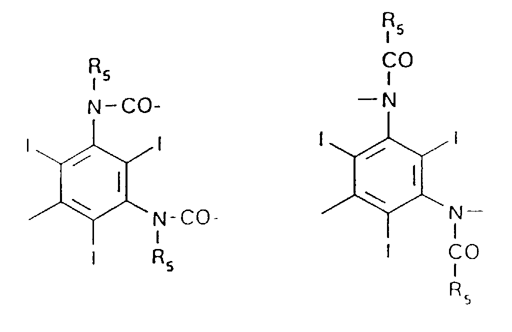 et 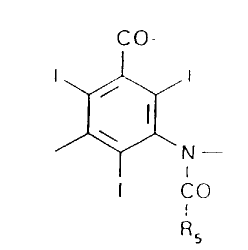 dans lesquels R₅ a les significations données ci-dessus et m représente un nombre entier de 2 à 12.

2. Composés polyiodés selon la revendication 1, caractérisés en ce que A est choisi parmi :
(i) un reste de formule CR₉(-R₁₀Y-)₃ dans laquelle R₉ représente un atome d'hydrogène, un groupe alkyle en C₁-C₆ ou aryle en C₅-C₁₀ ou le groupe (R₁₀-Y), R₁₀ étant choisi parmi les groupes alkylène (en C₁-C₆), arylène (en C₅-C₁₀), alkyl (en C₁-C₁₀) arylène (en C₅-C₁₀), et aryl (en C₅-C₁₀) -alkylène (en C₁-C₆), les groupes alkylène étant éventuellement interrompus par un ou plusieurs atomes d'oxygène, les groupes alkyle, alkylène, aryle ou arylène étant éventuellement substitués par un ou plusieurs groupes OH et Y étant choisi parmi les groupes -O-, -CO-, 〉N-R₅, 〉NCOR₅, R₅ étant tel que défini à la revendication 1;
(ii) un reste de cycloalcane en C₅ à C₁₂, éventuellement polycyclique, comportant éventuellement de 1 à 6 atomes d'iode, portant de 2 à 12 substituants identiques ou différents de formule -R₁₁(-Y-)_{q}, q représentant un entier de 1 à 3, R₁₁ étant une liaison simple ou un groupe alkylène linéaire ou ramifié en C₁-C₆ éventuellement substitué par un ou plusieurs groupes OH, et/ou interrompu par un ou plusieurs atomes d'oxygène, et Y étant tel que défini ci-dessus;
(iii) un reste aromatique hydrocarboné en C₅-C₁₂, monocyclique ou bicyclique, comportant éventuellement de 3 à 6 atomes d'iode et éventuellement un ou plusieurs substituants choisis parmi OH, NH₂, alkyle en C₁-C₆, hydroxy- ou polyhydroxyalkyle en C₁-C₆, COOH, COOR₁₂, -CO-NHR₁₂ ou -NR₆COR₅, tels que R₁₂ représente un groupe alkyle en C₁-C₄ et R₅ et R₆, identiques ou différents entre eux, représentent H, un groupe alkyle linéaire ou ramifié en C₁-C₁₀, hydroxy- ou polyhydroxyalkyle linéaire ou ramifié en C₁-C₁₀, alkoxy (en C₁-C₅) alkyle linéaire ou ramifié en C₁-C₁₀, hydroxy- ou polyhydroxy- alkoxy (en C₁-C₅) alkyle linéaire ou ramifié en C₁-C₁₀, ou R₅ et R₆ forment ensemble un groupe alkylène en C₄-C₈, hydroxy-alkylène en C₄-C₈ ou polyhydroxyalkylène en C₄-C₈, à chaîne linéaire ou ramifiée éventuellement interrompue par un ou plusieurs atomes choisis parmi S, O, P et N, de sorte que R₅ et R₆ forment avec l'atome d'azote auquel ils sont liés un hétérocycle azoté de 5 à 12 chaînons éventuellement substitué par un ou plusieurs groupes hydroxy, hydroxy- ou polyhydroxyalkyle en C₁-C₄ à chaîne linéaire ou ramifiée et renfermant éventuellement un ou plusieurs hétéroatomes supplémentaires choisis parmi S, O, P et N, ce reste comportant de 2 à 12 substituants de formule -R₁₁(-Y-)_{q}, Y, q et R₁₁ étant tels que définis précédemment;
(iv) un reste hétérocyclique éventuellement aromatique, monocyclique ou bicyclique, comportant de 5 à 10 chaînons dont 1 à 4 hétéroatomes choisis parmi O, S, N et P, éventuellement substitué par 3 à 6 groupes = O, ce reste comportant de 3 à 12 substituants de formule -R₁₁(-Y-)_{c}, R₁₁, Y et q étant tels que définis précédemment;
(v) un reste éventuellement cyclique comportant de 2 à 18 cycles aromatiques ou hétérocycliques tels que définis ci-dessus, liés entre eux par des groupes -R₁₁-, -OR₁₁-, ce reste comportant de 3 à 12 substituants de formule -R₁₁(-Y-)_{q}, R₁₁, Y et q étant tels que définis ci-dessus.

3. Composés polyiodés selon la revendication 1, caractérisés en ce que A est choisi parmi :
(i)- un reste de formule C(R₁₀-Y―)₁, R₁₀ représentant le groupe méthylène, phénylène, phénylméthylène ou le groupe -CH₂-O-CH₂- ou -CH₂-O-(CH₂)₂-, et Y étant tel que défini à la revendication 2 ;
(ii) - un reste de cycloalcane choisi parmi le cyclohexane et l'adamantane, substitués par 3 ou 4 substituants R₁₁―(Y―)_{q} tels que définis à la revendication 2;
(iii) - un groupe phényle éventuellement substitué par 3 à 4 atomes d'iode et un groupe biphényle éventuellement substitué par 4 à 6 atomes d'iode et comportant éventuellement de 3 à 6 substituants de formule R₁₁―(Y―)_{q}, R₁₁ et Y étant tels que définis à la revendication 2 et q représentant 1 à 4;
(iv) - un reste hétérocyclique constitué par le groupe 2,4,6-trioxo-1,3,5-triazine substitué sur les atomes d'azote par 3 substituants de formule -R₁₁―(Y―)_{q} telle que définie à la revendication 2 ;
(v) - un macrocycle composé de 6 à 8 résidus phényle chacun substitué par un ou plusieurs substituants choisis parmi OH, NH₂, alkyle en C₁-C₆, hydroxy- ou polyhydroxyalkyle en C₁-C₆, COOH, COOR₁₂, -CO-NHR₁₂ ou - NR₆COR₅, R₁₂ représentant un groupe alkyle en C₁-C₄, et un ou plusieurs groupes de formule R₁₁-(Y-)_{q} telle que définie ci-dessus avec q = 1, les résidus phényle étant liés entre eux par un groupe méthylène, ou une cyclodextrine composée de 6 à 8 résidus glucosyle liés entre eux par une liaison osidique, un ou plusieurs groupes OH desdits résidus glucosyle étant éventuellement substitués par un reste carboxyméthyle, un ou plusieurs groupes OH desdits résidus glucosyle étant remplacés par un groupe -O- ou un macrocyle polyazoté ou un dérivé d'un acide polyaminocarboxylique.

4. Composés polyiodés selon l'une des revendications 1 à 3, dans lesquels les groupes B₁ sont choisis parmi :

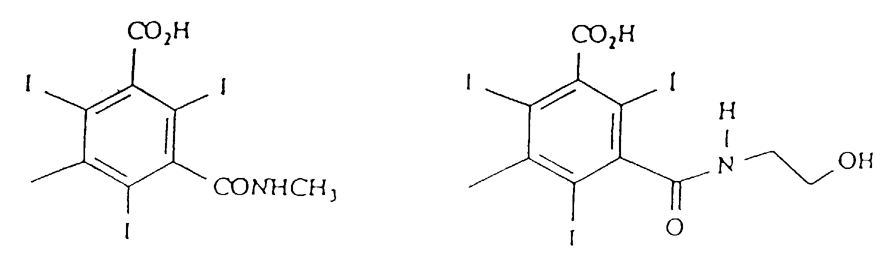

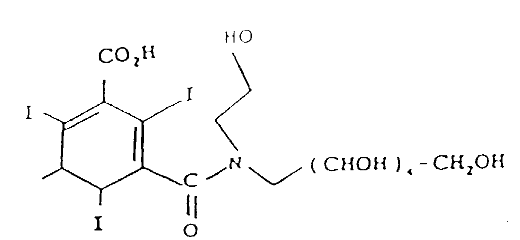

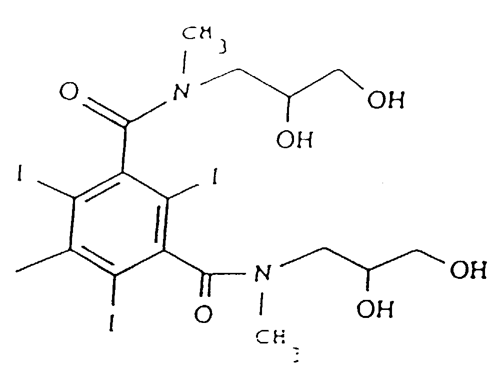

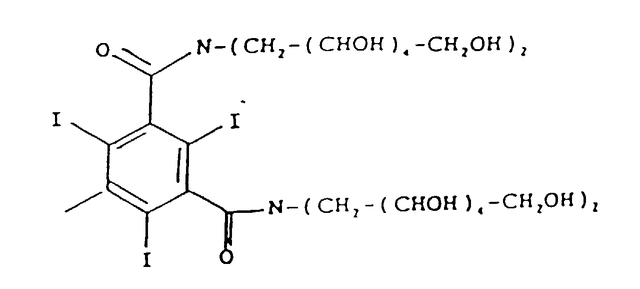

5. Composés polyiodés selon l'une des revendications 1 à 3, dans lesquels le groupe Q est choisi parmi : 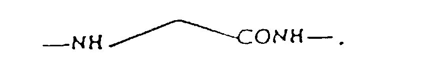 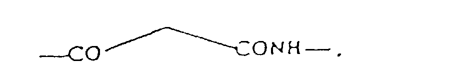 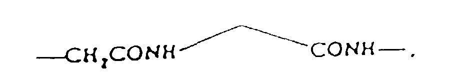
-(CH₂)ᵣCONH―,
et 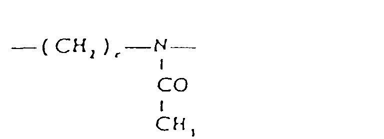 r étant un nombre entier de 1 à 5.

6. Composés polyiodés selon l'une des revendications 1 à 3, dans lesquels R₃ est choisi parmi : 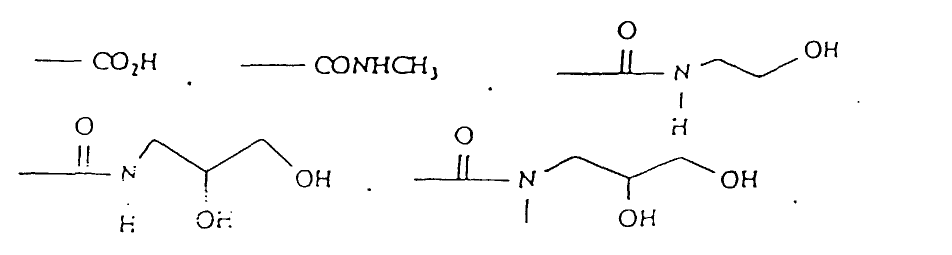 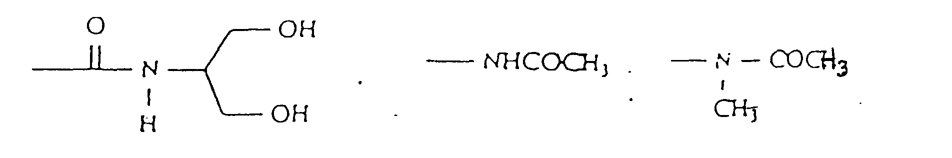 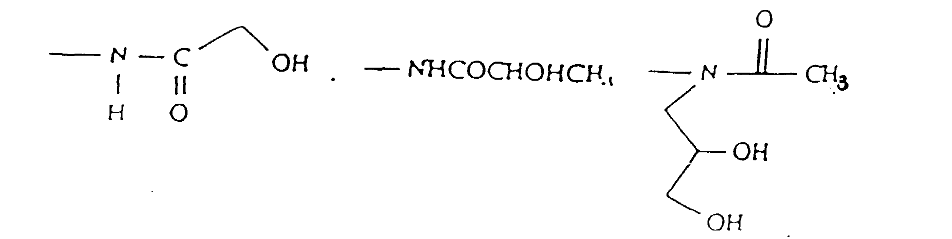 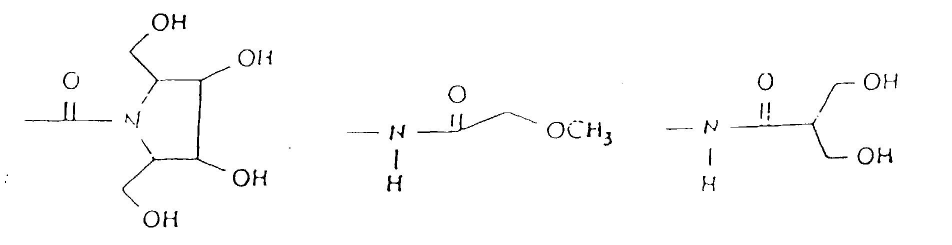 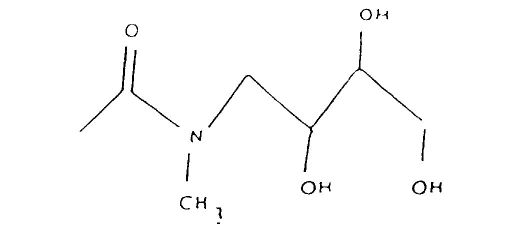 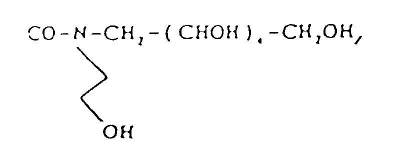 -CO-N-(CH₂-(CHOH)₄-CH₂OH)₂ -CO-NH-CH₂-C(CH₂-O-CH₂(CHOH)ₙ-CH₂OH)₃, n = 1 à 4 -CO-NH-CH₂-C(CH₂-O-CH₂CHOHCH₂OH)₃

7. Composés polyiodés selon l'une des revendications 1 à 3, dans lesquels R₄ représente un groupe R₃ ou un groupe de formule -Q-B₄, Q étant tel que défini à la revendication 1 et B₄ représentant un groupe de formule : 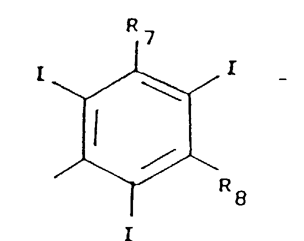 R₇ et R₈ ayant les mêmes significations que R₃.

8. Composés polyiodés selon les revendications 1 à 3, caractérisés en ce qu'ils répondent à la formule générale V : 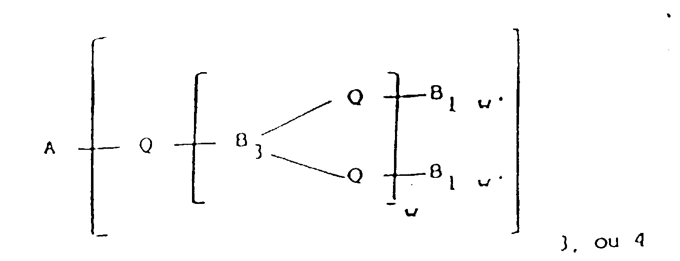 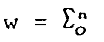 2ⁿ et w' = 2ⁿ (n représentant un nombre entier de 0 à 4)
dans laquelle A est tel que défini à la revendication 1, Q représente le groupe 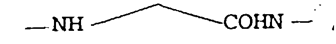 et B₁ représente le groupe : 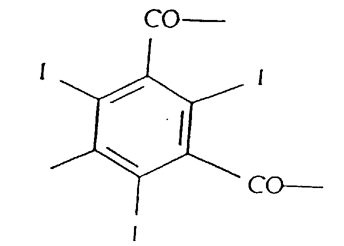 et B₁ représente le groupe 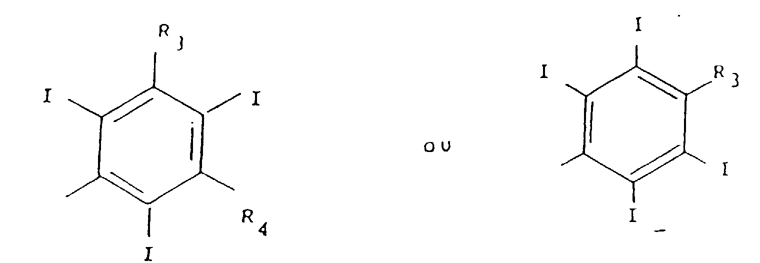 dans lesquelles
R₃ et R₄ sont choisis parmi CO⁻₂ M⁺ 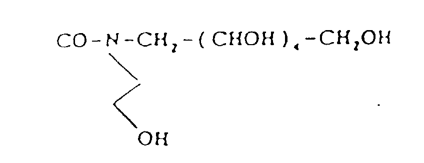 -CO-N-(CH₂-(CHOH)₄-CH₂OH)₂

9. Composés polyiodés selon l'une des revendications 1 à 3, caractérisés en ce qu'ils sont choisis parmi : 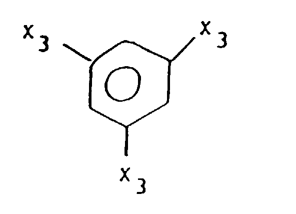 avec :

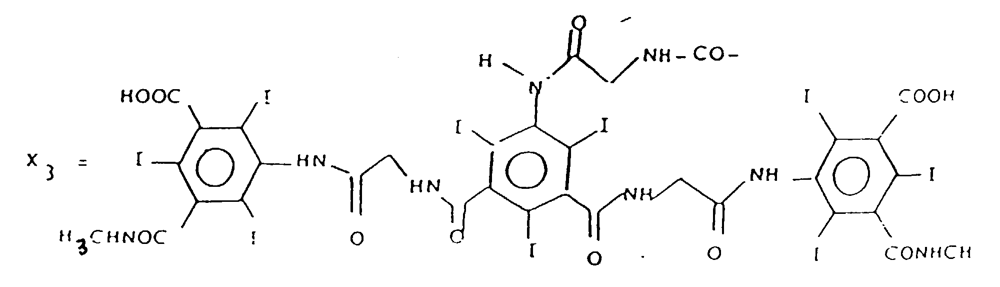

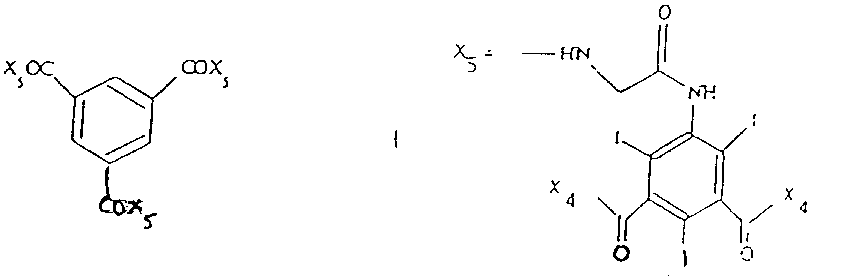

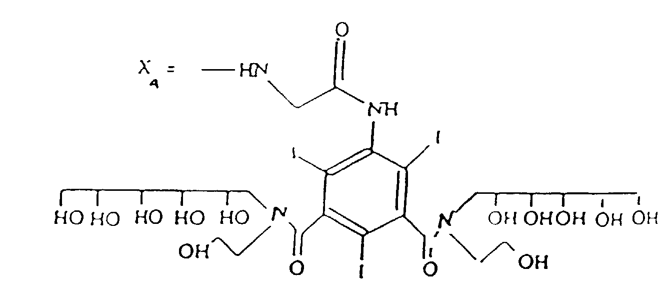

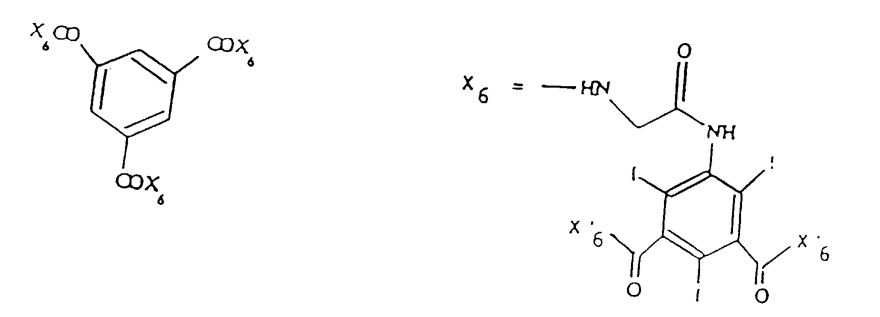

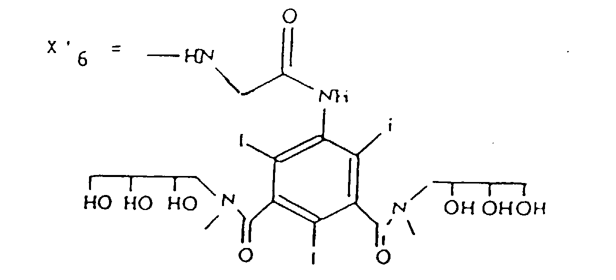

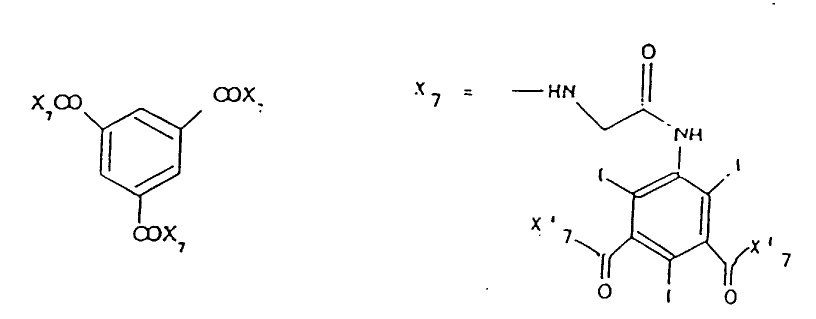

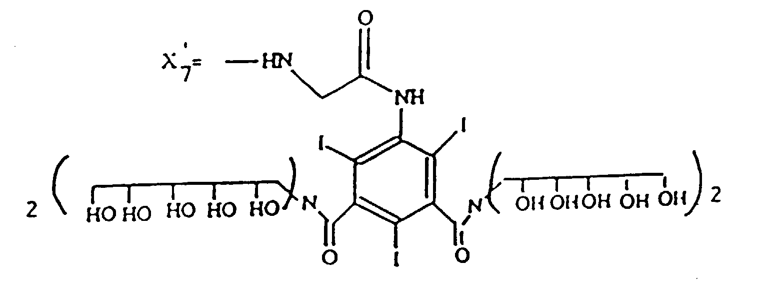

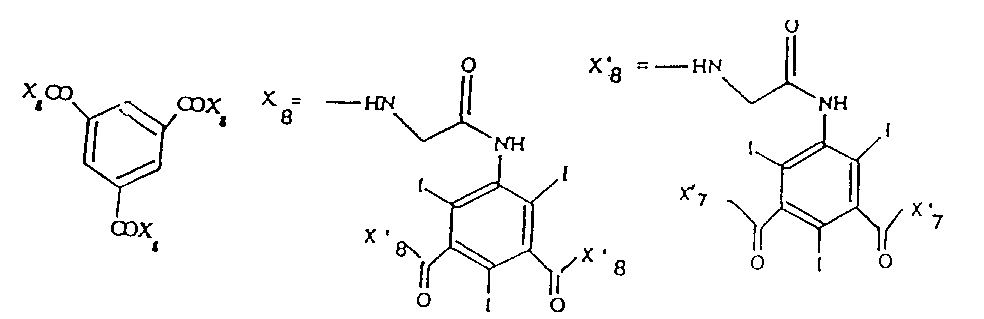

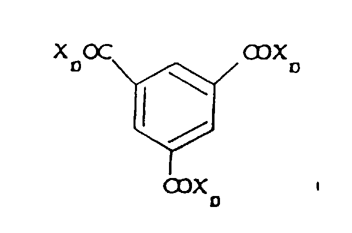

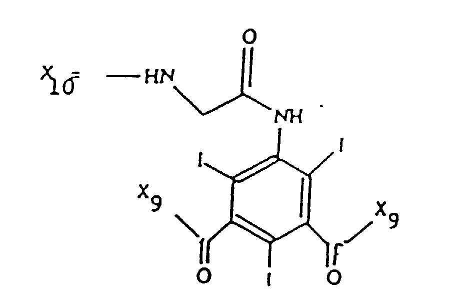

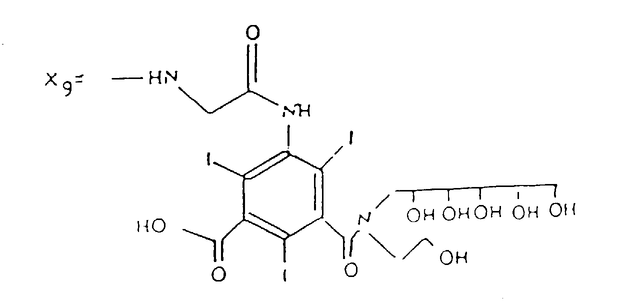

10. Produit de contraste, caractérisé en ce qu'il contient au moins un composé selon l'une quelconque des revendications 1 à 9.

11. Produit de contraste selon la revendication 10, caractérisé en ce qu'il est constitué par une solution aqueuse du ou des composés.

## Patentansprüche

1. Polyiodierte Verbindungen mit einem Molekulargewicht von mehr als 2000 und weniger als 50000 der allgemeinen Formel
A(̵X)ₘ
in der:
- A den Rest eines polyfunktionellen Moleküls, welcher ein zentrales tri- oder tetra-substituiertes Kohlenstoffatom, ein tri-substituiertes zentrales Stickstoff- oder Phosphoratom oder eine tri-substituierte Gruppe P=O und/oder mindestens einen gegebenenfalls aromatischen Kohlenstoffring, der gegebenenfalls ein oder mehrere Iodatome trägt, oder mindestens einen gegebenenfalls aromatischen Heterocyclus, der 1 bis 4 Heteroatome ausgewählt aus O, S, N und P aufweist, umfaßt, an dem m Gruppen X über m Bindungen oder m Gruppen ausgewählt aus -CO-, 〉N-R₅, -O-, 〉NCOR₅, worin R₅ H, eine geradkettige oder verzweigte C₁-C₁₀ -Alkylgruppe, eine geradkettige oder verzweigte C₁-C₁₀-Hydroxy- oder -Polyhydroxyalkylgruppe, eine geradkettige oder verzweigte C₁-C₁₀-Hydroxy- oder -Polyhydroxy-(C₁-C₅)-alkoxy-alkylgruppe darstellt, gebunden sind;
- die Gruppen X, die gleichartig oder verschieden sind: 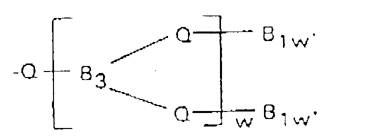 in der w = Σⁿₒ2ⁿ und w'= 2ⁿ darstellen, worin n eine ganze Zahl mit einem Wert von 0 bis 4 darstellt und die Gruppen Q, die untereinander gleichartig oder verschieden sind, eine Einfachbindung oder eine Gruppe ausgewählt aus: 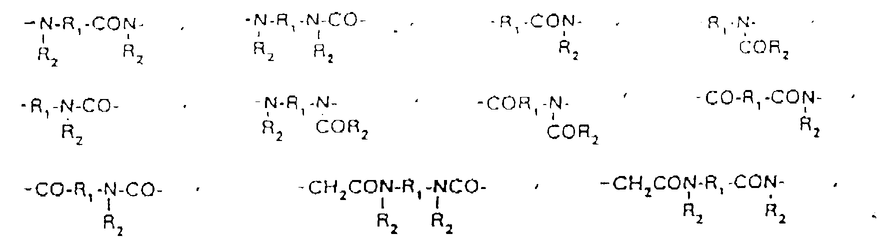 darstellen, worin R₁ aus einer Einfachbindung und geradkettigen oder verzweigten C₁-C₁₀-Alkylengruppen, geradkettigen oder verzweigten C₁-C₁₀-Hydroxy- oder -Polyhydroxyalkylengruppen, geradkettigen oder verzweigten (C₁-C₅)-Alkoxy-C₁-C₁₀-alkylengruppen oder geradkettigen oder verzweigten Hydroxy- oder Polyhydroxy-(C₁-C₅)-alkoxy-alkylengruppen, wobei die Alkylenkette gegebenenfalls durch ein oder mehrere Sauerstoffatome unterbrochen ist und die Gruppen R₂, die gleichartig oder verschieden sind, aus H, geradkettigen oder verzweigten C₁-C₁₀-Alkylgruppen, geradkettigen oder verzweigten C₁-C₁₀-Hydroxy- oder -Polyhydroxy-alkylgruppen, geradkettigen oder verzweigten (C₁-C₅)-Alkoxy-C₁-C₁₀-alkylgruppen und geradkettigen oder verzweigten Hydroxy- oder Polyhydroxy-(C₁-C₅)-alkoxy-alkylgruppen ausgewählt sind;
und die Gruppen B₁, die gleichartig oder verschieden sind, durch die folgenden Formeln: 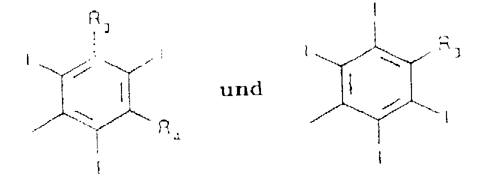 wiedergegeben werden, in denen R₃ aus den Gruppen -COO⁻M⁺, worin M⁺ H⁺ oder das physiologisch annehmbare Kation einer organischen oder anorganischen Base darstellt, 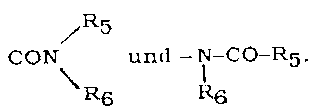 worin R₅ und R₆, die untereinander gleichartig oder verschieden sind, die oben für R₅ angegebenen Bedeutungen besitzen oder R₅ und R₆ gemeinsam eine C₄-C₈-Alkylengruppe, C₄-C₈-Hydroxyalkylengruppe oder C₄-C₈-Polyhydroxyalkylengruppe mit gerader oder verzweigter Kette, die gegebenenfalls durch ein oder mehrere Atome ausgewählt aus S, O, P und N unterbrochen sein kann, derart, daß R₅ und R₆ zusammen mit dem Stickstoffatom, an das sie gebunden sind, einen stickstoffhaltigen Heterocyclus mit 5 bis 12 Kettengliedern bilden, der gegebenenfalls durch eine oder mehrere Hydroxygruppen, geradkettige oder verzweigte C₁-C₄-Hydroxy- oder -Polyhydroxyalkylgruppen substituiert ist und gegebenenfalls ein oder mehrere zusätzliche Heteroatome ausgewählt aus S, O, P und N aufweist, R₄ eine Gruppe ausgewählt aus R₃ und Q-B₄ darstellt, worin die oben angegebenen Bedeutungen besitzt und B₄ eine Gruppe: 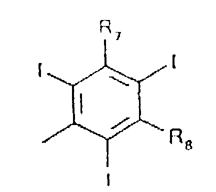 darstellt, in der R₇ und R₈, die untereinander gleichartig oder verschieden sind, R₃ darstellen, ausgewählt ist, und
die Gruppen B₃, die untereinander gleichartig oder verschieden sind, ausgewählt sind aus: 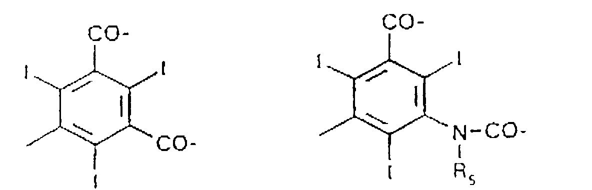 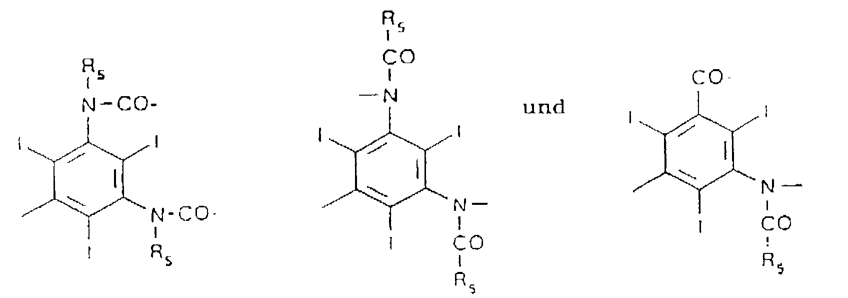 worin R₅ die oben angegebenen Bedeutungen besitzt und
m für eine ganze Zahl mit einem Wert von 2 bis 12 steht,
bedeuten.

2. Polyiodierte Verbindungen nach Anspruch 1, dadurch gekennzeichnet, daß A ausgewählt ist aus:
(i) einem Rest der Formel CR₉(-R₁₀Y-)₃, in der R₉ ein Wasserstoffatom, eine C₁-C₆-Alkylgruppe oder eine C₅-C₁₀-Arylgruppe oder die Gruppe (R₁₀-Y) darstellt, worin R₁₀ aus (C₁-C₆)-Alkylengruppen, (C₅-C₁₀)-Arylengruppen, (C₁-C₁₀)-Alkyl-(C₅-C₁₀)-arylengruppen und (C₅-C₁₀)-Aryl-(C₁-C₆)-alkylengruppen ausgewählt ist, wobei die Alkylengruppen gegebenenfalls durch ein oder mehrere Sauerstoffatome unterbrochen sind und die Alkyl-, Alkylen-, Aryl- oder Arylengruppen gegebenenfalls durch eine oder mehrere Gruppen OH substituiert sind, und Y ausgewählt ist aus den Gruppen -O-. -CO-, 〉N-R₅, 〉NCOR₅, worin R₅ die in Anspruch 1 angegebenen Bedeutungen besitzt;
(ii) einem C₅-C₁₂-Cycloalkanrest, der gegebenenfalls polycyclisch ist und gegebenenfalls 1 bis 6 Iodatome trägt, 2 bis 12 gleichartige oder verschiedene Substituenten der Formel -R₁₁(-Y-)_{q} trägt, in der q eine ganze Zahl mit einem Wert von 1 bis 3 darstellt, R₁₁ eine Einfachbindung oder eine geradkettige oder verzweigte C₁-C₆-Alkylengruppe darstellt, die gegebenenfalls durch eine oder mehrere OH-Gruppen substituiert ist, und/oder die durch ein oder mehrere Sauerstoffatome unterbrochen ist, während Y die oben angegebenen Bedeutungen besitzt;
(iii) einem monocyclischen oder bicyclischen aromatischen C₅-C₁₂-Kohlenwasserstoffrest, der gegebenenfalls 3 bis 6 Iodatome und gegebenenfalls einen oder mehrere Substituenten ausgewählt äus OH, NH₂, C₁-C₆-Alkyl, C₁-C₆-hydroxy- oder -Polyhydroxyalkyl, COOH, COOR₁₂, -CO-NHR₁₂ oder -NR₆COR₅ trägt, worin R₁₂ einc C₁-C₄-Alkylgruppe darstellt und R₅ und R₆, die untereinander gleichartig oder verschieden sind, H, eine geradkettige oder- verzweigte C₁-C₁₀-Alkylgruppe, eine geradkettige oder verzweigte C₁-C₁₀-Hydroxy- oder -Polyhydroxyalkylgruppe, eine geradkettige oder verzweigte (C₁-C₅)-Alkoxy-C₁-C₁₀-alkylgruppe, eine geradkettige oder verzweigte (C₁-C₅)-Alkoxy-C₁-C₁₀-alkylgruppe darstellen oder R₅ und R₆ gemeinsam eine C₄-C₈-Alkylengruppe, eine C₄-C₈-Hydroxyalkylen- oder C₄-C₈-Polyhydroxyalkylengruppe mit gerader oder verzweigter Kette bilden, die gegebenenfalls durch ein oder mehrere Atome ausgewählt aus S, O. P und N unterbrochen ist, derart, daß R₅ und R₆ zusammen mit dem Stickstoffatom, an das sie gebunden sind, einen stickstoffhaltigen Heterocyclus mit 5 bis 12 Kettengliedern bilden, der gegebenenfalls durch eine oder mehrere Hydroxygruppen, geradkettige oder verzweigte C₁-C₄-Hydroxy- oder -Polyhydroxyalkylgruppen substituiert ist und gegebenenfalls ein oder mehrere zusätzliche Heteroatome ausgewählt aus S, O, P und N aufweist, wobei dieser Rest 2 bis 12 Substituenten der Formel -R₁₁(-Y-)_{q} trägt, worin Y, q und R₁₁ die oben angegebenen Bedeutungen besitzen:
(iv) einem monocyclischen oder bicyclischen, gegebenenfalls aromatischen heterocyclischen Rest, der 5 bis 10 Kettenglieder aufweist, darunter 1 bis 4 Heteroatome ausgewählt aus O, S, N und P, der gegebenenfalls durch 3 bis 6 Gruppen =O substituiert ist, wobei dieser Rest 3 bis 12 Substituenten der Formel -R₁₁(-Y-)_{q} aufweist, worin R₁₁, Y und q die oben angegebenen Bedeutungen besitzen;
(v) einem gegebenenfalls cyclischen Rest, der 2 bis 18 aromatische oder heterocyclische Ringe aufweist, wie sie oben definiert sind, die untereinander durch Gruppen -R₁₁-, -OR₁₁- verbunden sind, wobei dieser Rest 3 bis 12 Substituenten der Formel -R₁₁(-Y-)_{q} aufweist, worin R₁₁, Y und q die oben angegebenen Bedeutungen besitzen.

3. Polyiodierte Verbindungen nach Anspruch 1, dadurch gekennzeichnet, daß A ausgewählt ist aus:
(i) - einem Rest der Formel C(R₁₀-Y-)₄, in der R₁₀ für die Methylengruppe, Phenylengruppe, Phenylmethylengruppe oder die Gruppe -CH₂-O-CH₂- oder -CH₂-O-(CH₂)₂- steht und Y die in Anspruch 2 angegebenen Bedeutungen besitzt;
(ii) - einem Cycloalkanrest ausgewählt aus Cyclohexan und Adamantan, der durch 3 oder 4 Substituenten R₁₁-(Y-)_{q} substituiert ist, wie sie in Anspruch 2 definiert sind;
(iii) - eine Phenylgruppe die gegebenenfalls durch 3 bis 4 Iodatome substituiert ist und eine Biphenylgruppe, die gegebenenfalls durch 4 bis 6 Iodatome substituiert ist und gegebenenfalls 3 bis 6 Substituenten der Formel R₁₁-(Y-)_{q} aufweist, worin R₁₁ und Y die in Anspruch 2 angegebenen Bedeutungen besitzen und q 1 bis 4 darstellt;
(iv) - einem heterocyclischen Rest, der durch die Gruppe 2,4,6-Trioxo-1,3,5-triazin gebildet ist, der an den Stickstoffatomen durch 3 Substituenten der Formel -R₁₁-(Y-)_{q}, wie sie in Anspruch 2 definiert ist, substituiert ist;
(v) - einem Makrocyclus, der aus 6 bis 8 Phenylresten gebildet ist, von denen jeder durch einen oder mehrere Substituenten ausgewählt aus OH. NH₂, C₁-C₆-Alkyl, C₁-C₆-Hydroxy- oder -Polyhydroxyalkyl, COOH, COOR₁2, -CO-NHR₁₂ oder -NR₆COR₅, worin R₁₂ für eine C₁-C₄-Alkylgruppe steht, und eine oder mehrere Gruppen der Formel R₁₁-(Y-)_{q}, wie sie oben definiert worden ist, worin q 1 bedeutet, substituiert ist, wobei die Phenylreste untereinander durch eine Methylengruppe oder eine Cyclodextringruppe aus 6 bis 8 Glucosylresten, die untereinander über eine osidische Gruppe verbunden sind, verbunden sind, wobei eine oder mehrere OH-Gruppen der Glucosylreste gegebenenfalls durch einen Carboxymethylrest substituiert sind oder eine oder mehrere Gruppen OH der Glucosylreste durch eine Gruppe -O- oder einen Polyazomakrocyclus oder ein Derivat einer Polyaminocarbonsäure ersetzt sind.

4. Polyiodierte Verbindungen nach einem der Ansprüche 1 bis 3, worin die Gruppen B₁ ausgewählt sind aus:

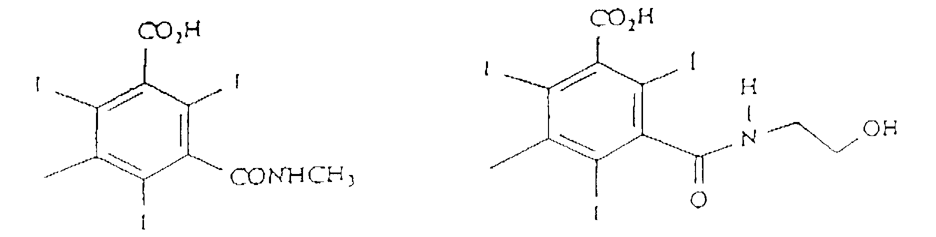

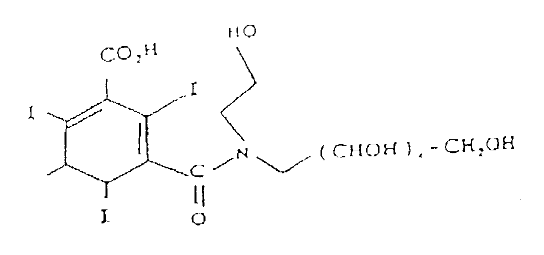

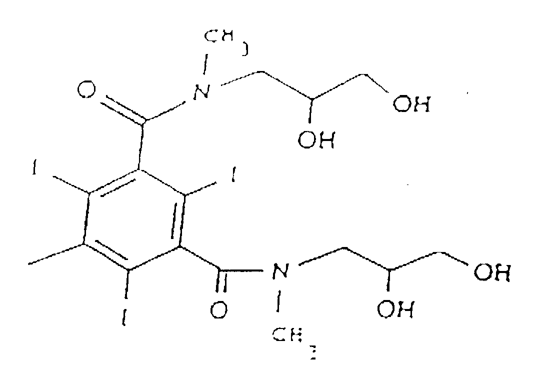

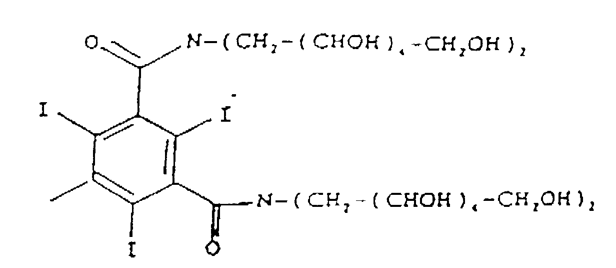

5. Polyiodierte Verbindungen nach einem der Ansprüche 1 bis 3, worin die Gruppe Q ausgewählt ist aus: 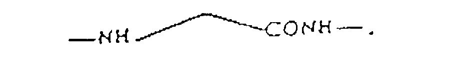 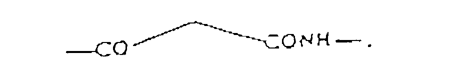 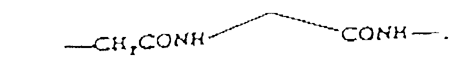
―(CH₂)ᵣCONH―
und 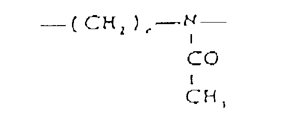 in denen r eine ganze Zahl mit einem Wert von 1 bis 5 darstellt.

6. Polyiodierte Verbindungen nach einem der Ansprüche 1 bis 3, worin R₃ ausgewählt ist aus: 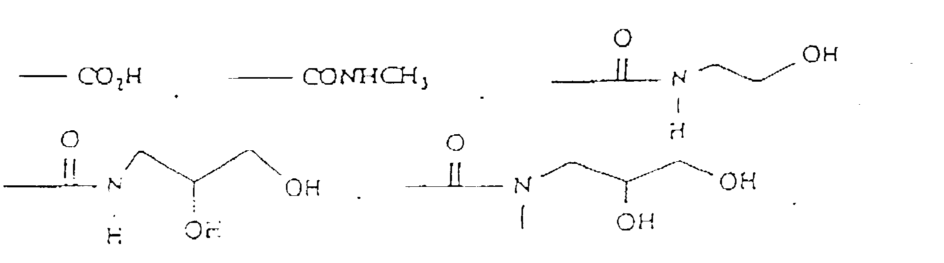 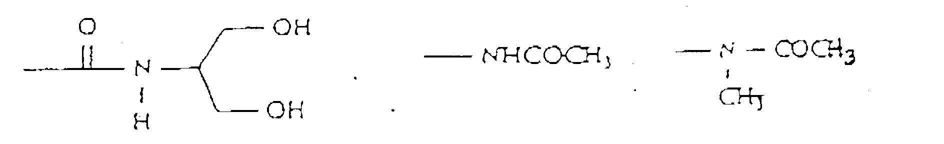 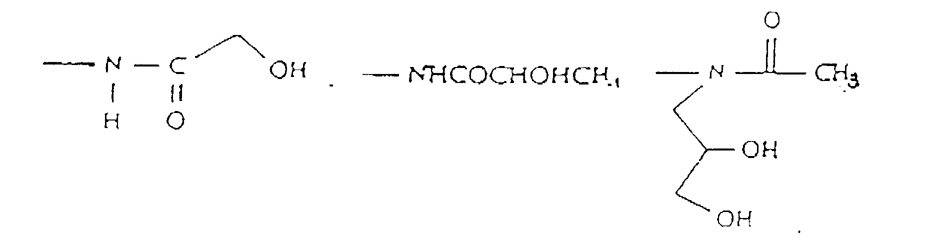 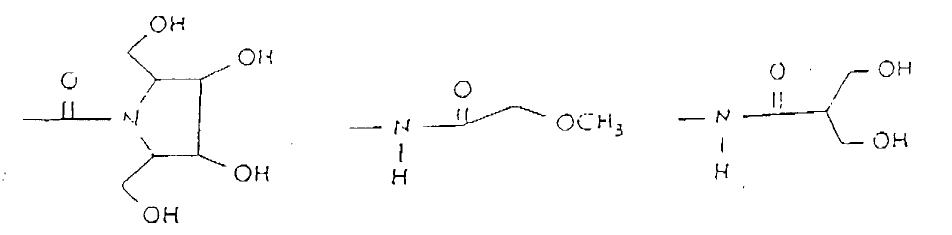 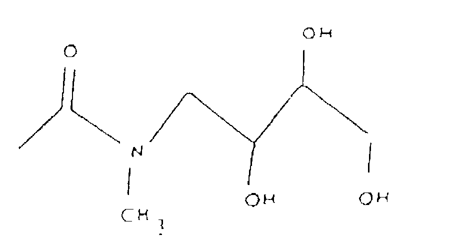 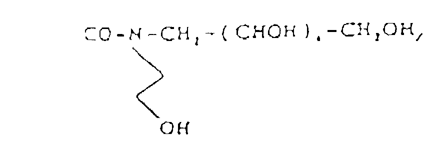 -CO-N-(CH₂-(CHOH)₄-CH₂OH)₂ -CO-NH-CH₂-C(CH₂-O-CH₂(CHOH)ₙ-CH₂OH)₃, n = 1 bis 4 -CO-NH-CH₂-C(CH₂-O-CH₂CHOHCH₂OH)₃

7. Polyiodierte Verbindungen nach einem der Ansprüche 1 bis 3, worin R₄ für eine Gruppe R₃ oder eine Gruppe der Formel -Q-B₄ steht, worin Q die in Anspruch 1 angegebenen Bedeutungen besitzt und B₄ eine Gruppe der Formel: 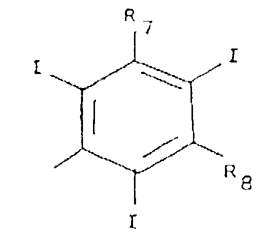 darstellt, in der R₇ und R₈ die gleichen Bedeutungen besitzen wie R₃.

8. Polyiodierte Verbindungen nach den Ansprüchen 1 bis 3, dadurch gekennzeichnet, daß sie der allgemeinen Formel V entsprechen: 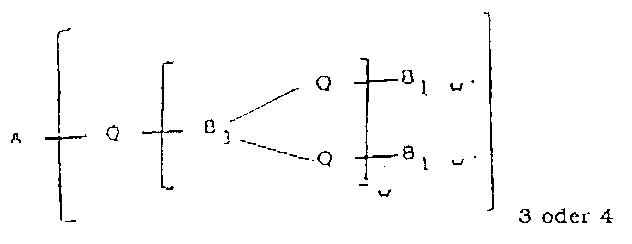 in der w = Σⁿₒ 2ⁿ und w' = 2ⁿ (worin n für eine ganze zahl mit einem Wert von 0 bis 4 steht),
worin A die in Anspruch 1 angegebenen Bedeutungen besitzt,
Q die Gruppe 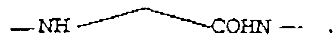 und B₃ die Gruppe: 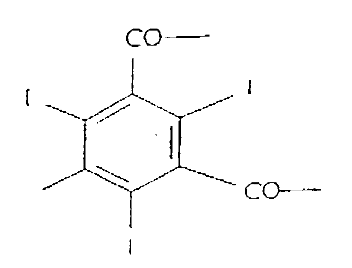 und B₁ die Gruppe 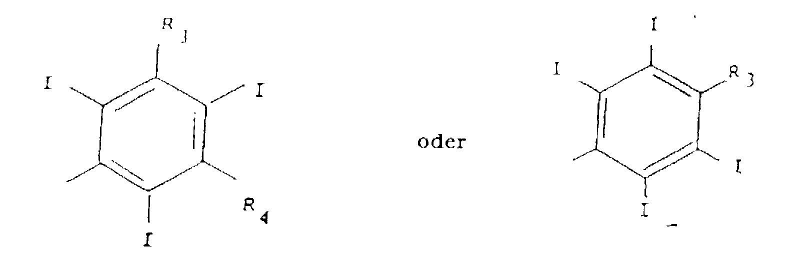 darstellen, worin R₃ und R₄ aus CO⁻₂ M⁺ 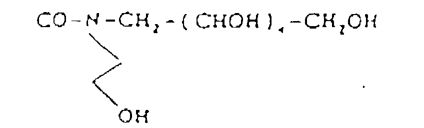 -CO-N-(CH₂-(CHOH)₄-CH₂OH)₂ ausgewählt sind.

9. Polyiodierte Verbindungen nach einem der Ansprüche 1 bis 3, dadurch gekennzeichnet, daß sie ausgewählt sind aus: 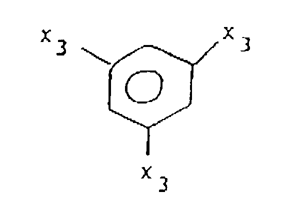 in der:

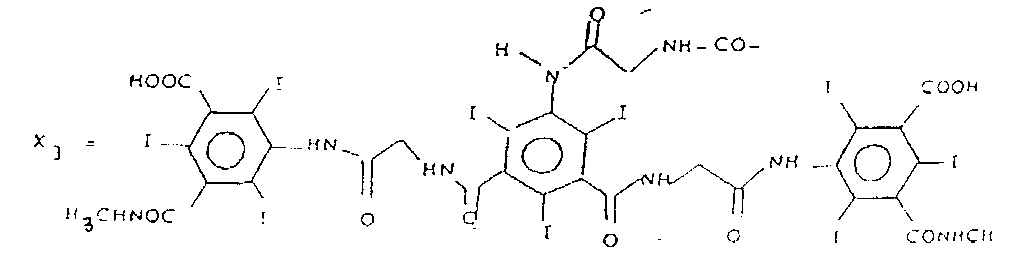

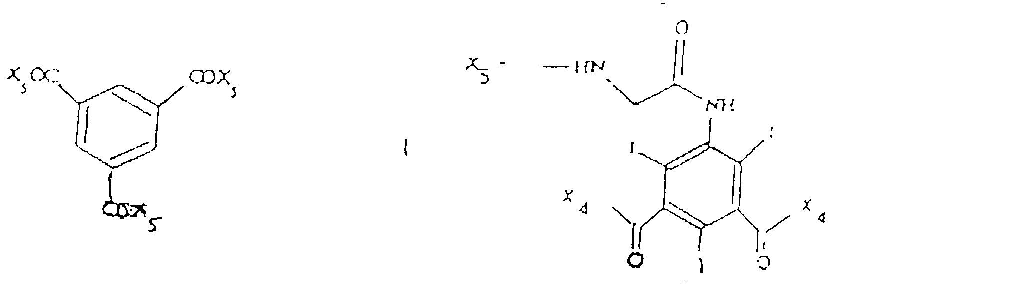

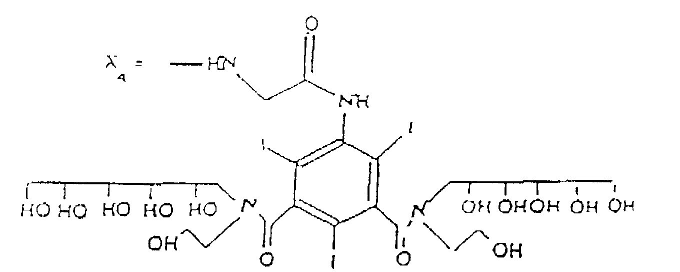

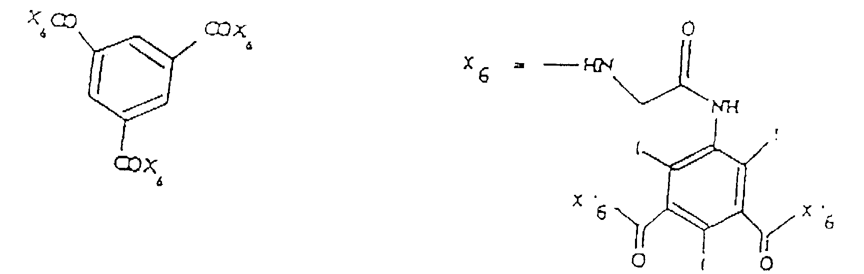

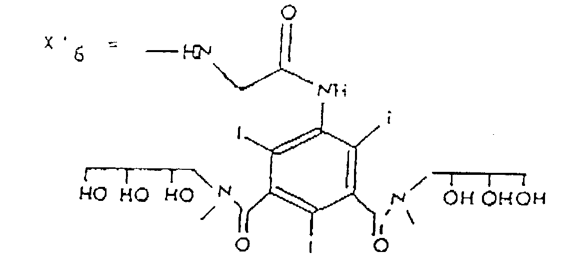

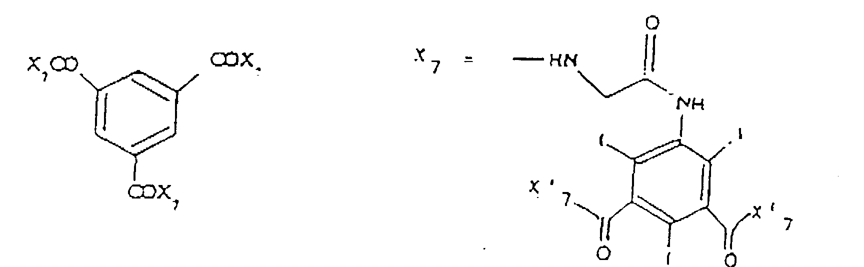

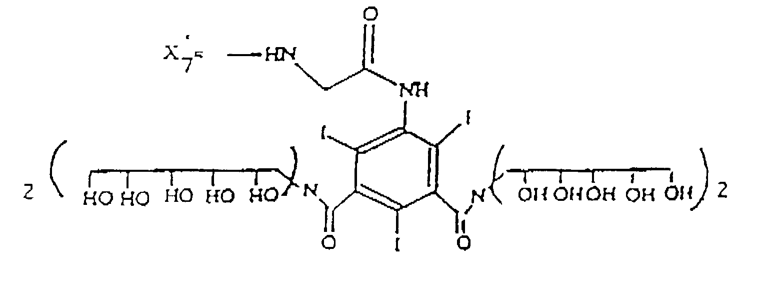

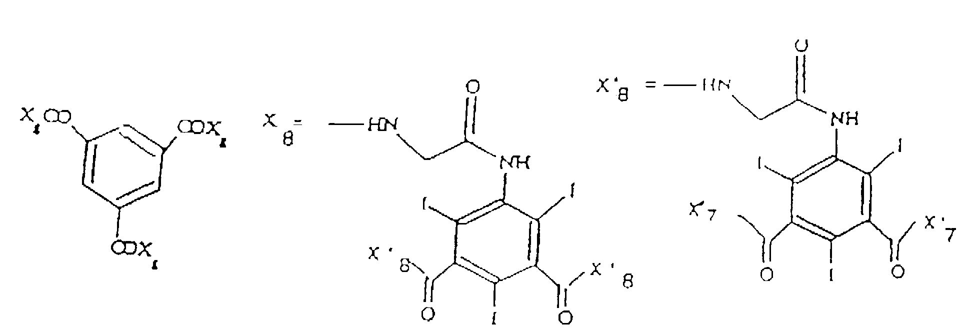

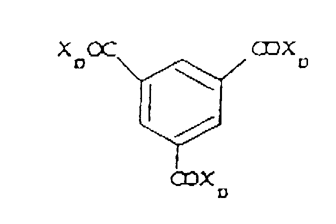

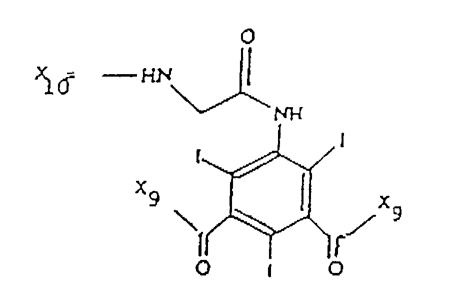

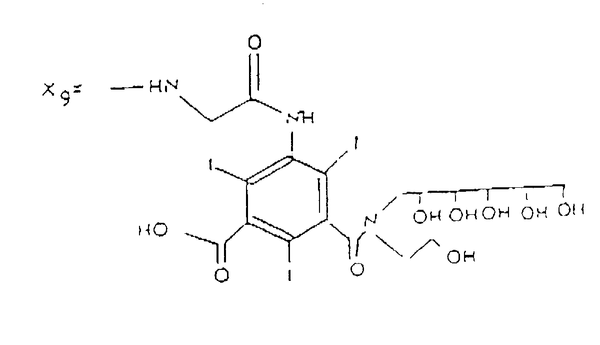

10. Kontrastmittel, dadurch gekennzeichnet, daß es mindestens eine Verbindung nach irgendeinem der Ansprüche 1 bis 9 enthält.

11. Kontrastmittel nach Anspruch 10, dadurch gekennzeichnet, daß es aus einer wäßrigen Lösung der Verbindung(en) gebildet ist.

## Claims

1. Polyiodinated compounds having a molecular weight greater than 2000 and less than 50000, of the general formula
A-(-X)ₘ
in which:
- A represents the residue of a poly-functional molecule comprising a tri- or tetra-substituted central carbon atom, a tri-substituted central atom of nitrogen or phosphorus or a tri-substituted P=O group and/or at least one aromaticor non-aromatic carbon ring, optionally comprising one or more iodine atoms, or at least one aromatic or non-aromatic hetero-ring comprising from I to 4 hetero-atoms selected from O, S, N and P, to which are attached the m groups X by way of m bonds or of m groups selected from -OC-, 〉N-R₅, -O-, 〉NCOR₅, with R₅ representing H, a straight chain or branched alkyl group from C₁ to C₁₀, a straight chain or branched hydroxy- or poly-hydroxy-alkyl from C₁ to C₁₀, straight chain or branched alkoxy- (from C₁ to C₅) alkyl from C₁ to C₁₀, straight chain or branched hydroxy- or poly-hydroxy-alkoxy (from C₁ to C₅) alkyl from C₁ to C₁₀;
- the groups X, which are the same or different from each other, representing: 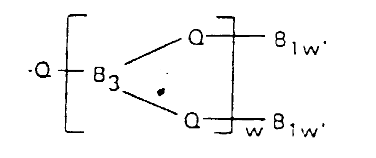 *with* 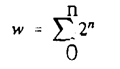 and w' = 2ⁿ, n representing an integer from 0 to 4 and the groups Q, which are the same or different from each other, representing a single bond or a group selected from: 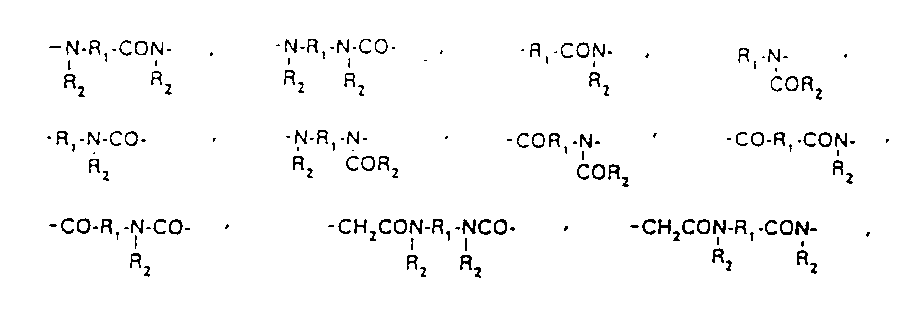 in which R₁ is selected from a single bond and the straight chain or branched alkylene groups with C₁ to C₁₀, hydroxy or poly-hydroxy straight chain or branched alkylene with C₁ to C₁₀, alkoxy (with C₁ to C₅) straight chain or branched alkylene with C₁ to C₁₀, hydroxy- or poly-hydroxy-alkoxy (with C₁ to C₅) straight chain or branched alkylene, the alkylene chain being optionally interrupted by one or more atoms of oxygen the groups R₂, which are the same or different from each other, are selected from H, the straight chain or branched alkyl groups with C₁ to C₁₀, hydroxy- or poly-hydroxy-alkyl, straight chain or branched with C₁ to C₁₀, alkoxy (with C₁ to C₅) straight chain or branched alkyl with C₁ to C₁₀, hydroxy- or poly-hydroxy-alkoxy (with C₁ to C₅) straight chain or branched alkyl;
and the groups B₁, which are the same or different from each other, are represented by the formulae: 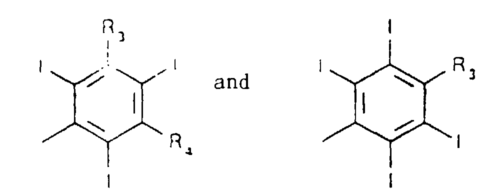 in which R₃ is selected from the groups -COOM⁺, with M⁺ representing H⁺ or the physiologically acceptable cation of an organic or mineral base, 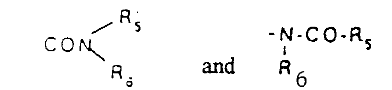 in which R₅ and R₆, the same or different from one another, are defined as above for R₅ or R₅ and R₆ forming together an alkylene group with C₄ to C₈ hydroxyalkylene with C₄ to C₈ or poly-hydroxylalkylene with C₄ to C₈, with a straight or branched chain optionally interrupted by one or more atoms selected from S, O, P and N, such that R₅ and R₆ form with the nitrogen atom to which they are bound a nitrogenous hetero-ring of 5 to 12 chain links optionally substituted by one or more hydroxy, hydroxy- or poly-hydroxy- straight chain or branched alkyl with C₁ to C₄ groups and optionally including one or more supplementary hetero atoms selected from S, O, P and N, R₄ representing a group selected from R₃ and Q-B₄, Q being as defined above and B₄ representing a group: 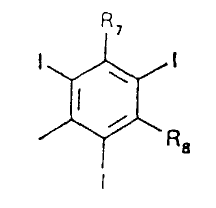 in which R₇ and R₈, the same or different from one another, represent R₃,
and the groups B₃, the same or different from another, are selected from: 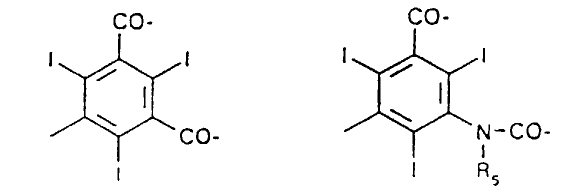 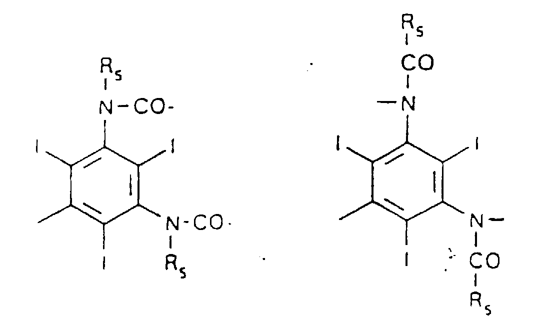 and 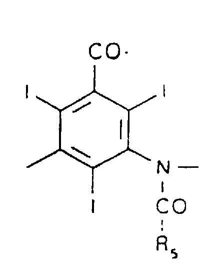 in which R₅ has the meanings given above
and m represents an integer from 2 to 12.

2. Polyiodinated compounds according to claim 1, characterized in that A is selected from:
(i) a residue of the formula CR₉(-R₁₀Y-)₃, in which R₉ represents an atom of hydrogen, an alkyl group with C₁ to C₆ or an aryl group with C₅ to C₁₀ or the group (R₁₀-Y), R₁₀ being selected from the alkylene (with C₁ to C₆), arylene (with C₅ to C₁₀), alkyl (with C₁ to C₁₀) arylene (with C₅ to C₁₀), and aryl (with C₅ to C₁₀) -alkylene (with C₁ to C₆) groups, the alkylene groups being optionally interrupted by one or more atoms of oxygen, the alkyl, alkylene, aryl or arylene groups being optionally substituted by one or more OH and Y being selected from the groups -O-, -CO-, 〉N-R₅, 〉NCOR₅, R₅ being as defined in claim 1;
(ii) a cyclo-alkane residue with C₅ to C₁₂, optionally polycyclic, optionally comprising I to 6 atoms of iodine, carrying 2 to 12 substituents which are the same or different of the formula -R₁₁(-Y-)_{q}, q being an integer from 1 to 3, R₁₁ being a single bond or a straight chain or branched alkylene group with C₁ to C₆ optionally substituted by one or more OH groups, and/or interrupted by one or more atoms of oxygen, and Y being as defined above;
(iii) a monocyclic or bicyclic aromatic hydrocarbon residue with C₅ to C₁₂, optionally comprising 3 to 6 atoms of iodine and optionally one or more substituents selected from OH, NH₂, alkyl with C₁ to C₆, hydroxy- or poly-hydroxy-alkyl with C₁ to C₆, COOH, COOR₁₂, -CO-NHR₁₂ or -NR₆COR₅, such that R₁₂ represents an alkyl group with C₁ to C₄ and R₅ and R₆, the same or different from one another, representing H, a straight chain or branched alkyl group with C₁ to C₁₀, straight chain or branched hydroxy- or poly-hydroxy-alkyl with C₁ to C₁₀, straight chain or branched alkoxy (with C₁ to C₅) alkyl with C₁ to C₁₀, hydroxy- or poly-hydroxy- alkoxy (with C₁ to C₅) alkyl, straight chain or branched, with C₁ to C₁₀, or R₅ and R₆ together forming an alkylene group with C₄ to C₈, a hydroxy alkylene group with C₄ to C₈ or a poly-hydroxy alkylene group with C₄ to C₈, with a straight chain or branched chain, optionally interrupted by one or more atoms selected from S, O, P and N, such that R₅ and R₆ form a nitrogen hetero-ring with the atom of nitrogen to which they are linked, with 5 to 12 links optionally substituted by one or more hydroxy, hydroxy- or poly-hydroxy- alkyl groups with C₁ to C₄ with a straight chain or branched, and optionally including one or more supplementary hetero atoms selected from S, O, P and N, this residue compnsing 2 to 12 substituents of the formula -R₁₁(-Y-)_{q}, Y, q and R₁₁ being as defined above;
(iv) a heterocyclic residue, optionally aromatic, monocyclic or bicyclic, comprising 5 to 10 links of which there are 1 to 4 hetero atoms selected from O, S, N and P, optionally substituted by 3 to 6=O groups, this residue comprising 3 to 12 substituents of the formula -R₁₁(-Y-)_{q}, R₁₁, Y and q being as defined above;
(v) an optionally cyclic residue compnsing 2 to 18 aromatic or heterocyclic rings such as defined above connected together by groups -R₁₁-, -OR₁₁-, this residue comprising 3 to 12 substituents of the formula R₁₁(-Y-)_{q}, Y, q and R₁₁ being as defined above.

3. Polyiodinated compounds according to claim 1, characterized in that A is selected from:
(i) a residue of the formula C(R₁₀-Y-)₄, R₁₀ representing the methylene, phenylene, phenylmethylene group or the group -CH₂-O-CH₂- or -CH₂-O-(CH₂)₂⁻ and Y being as defined in claim 2;
(ii) a cyclo-alkane residue selected from cyclohexane and adamantane, substituted by 3 or 4 substituents R₁₁-(Y-)_{q} as defined in claim 2;
(iii) a phenyl group optionally substituted by 3 to 4 atoms of iodine and a biphenyl group optionally substituted by 4 to 6 atoms of iodine and optionally comprising 3 to 6 substituents of the formula R₁₁-(Y-)_{q}, R₁₁ and Y being as defined in claim 2 and q representing 1 to 4;
(iv) a heterocyclic residue formed by the group 2,4,6-trioxo-1,3,5-triazine substituted on the atoms of nitrogen by 3 substituents of the formula -R₁₁-(Y-)_{q} as defined in claim 2;
(v) a macro-ring composed of 6 to 8 phenyl residues, each substituted by one of more substituents selected from OH, NH₂, alkyl with C₁ to C₆, hydroxy- or poly-hydroxy-alkyl with C₁ to C₆, COOH, COOR₁₂, -CO-NHR₁₂ or -NR₆COR₅, R₁₂ representing an alkyl group with C₁ to C₄, and one or more groups of the formula R₁₁-(Y-)_{q} as defined above with q = 1, the phenyl residues being linked to one another by a methylene group, or a cyclodextrin comprising 6 to 8 glycosyl residues linked to one another by an osidic bond, one or more OH groups of the said glycosyl residues being optionally substituted by a carboxymethyl residue, and one or more OH groups of the said glycosyl; residues being replaced by an -O- group or a poly-nitrogenous macro-ring or a derivative of a polyaminocarboxylic acid.

4. Polyiodinated compounds according to any of claims 1 to 3, in which the groups B₁ are selected from:

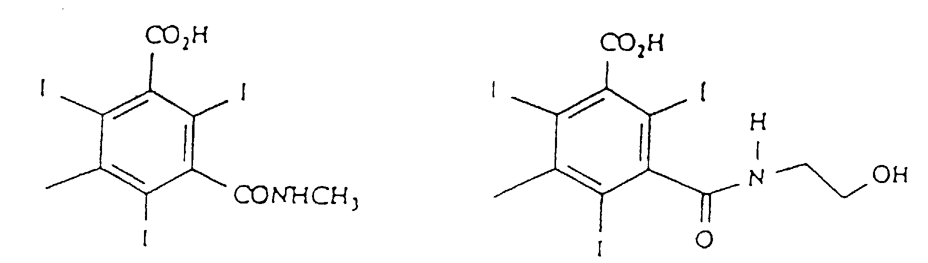

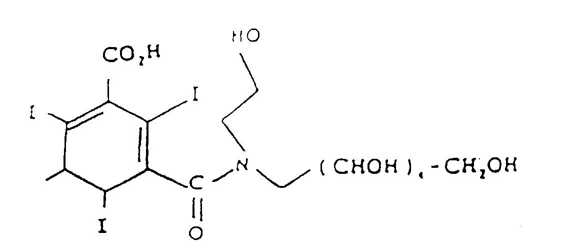

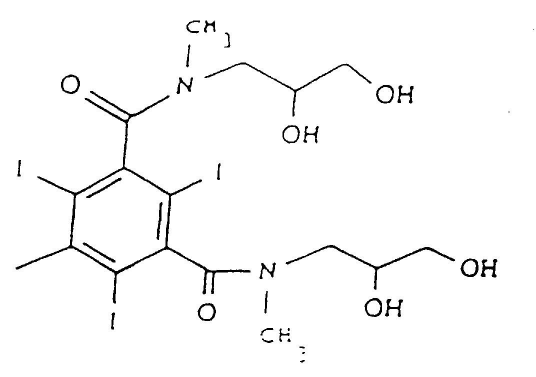

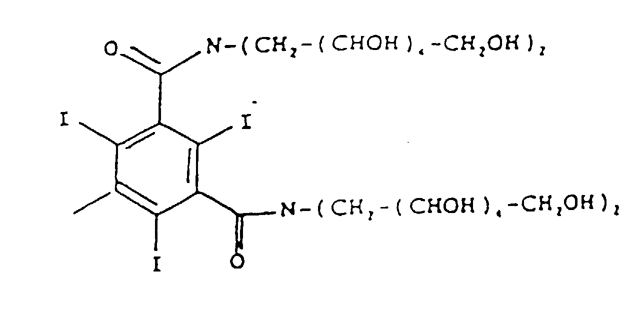

5. Polyiodinated compounds according to any of claims 1 to 3, in which the group Q is selected from: 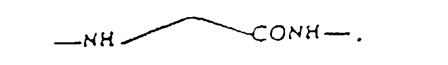 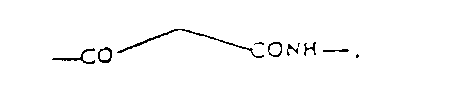 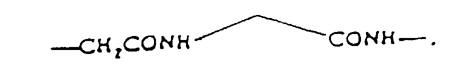
―(CH₂)ᵣCONH―,
and 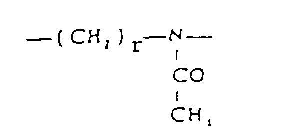 r being an integer from 1 to 5.

6. Polyiodinated compounds according to any of claims 1 to 3, in which R₃ is selected from: 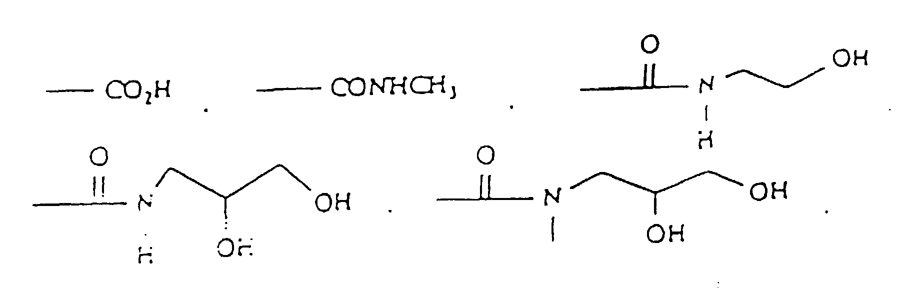 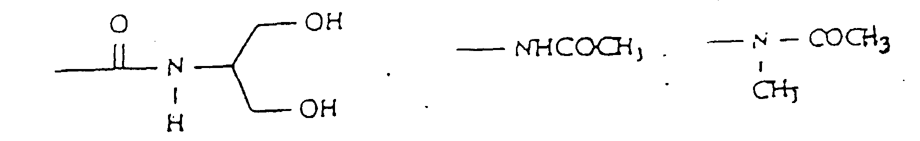 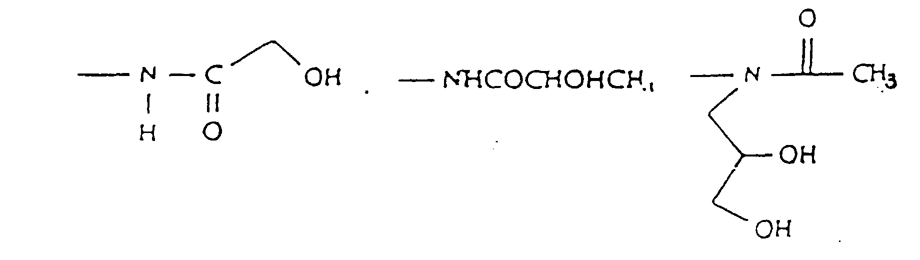 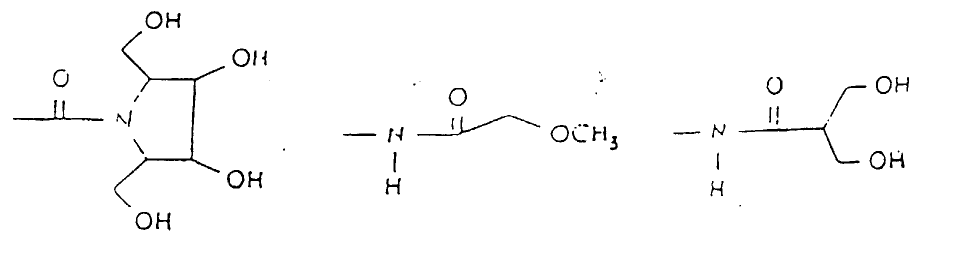 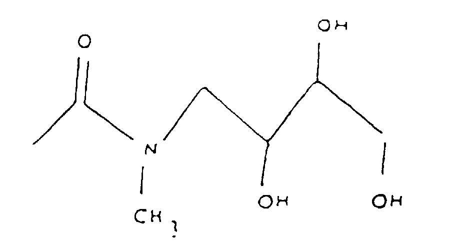 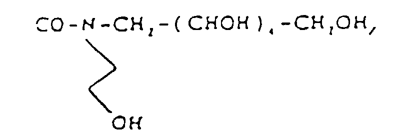 -CO-N-(CH₂-(CHOH)₄-CH₂OH)₂ -CO-NH-CH₂-C(CH₂-O-CH₂(CHOH)ₙ-CH₂OH)₃, n = 1 to 4 -CO-NH-CH₂-C(CH₂-O-CH₂CHOHCH₂OH)₃

7. Polyiodinated compounds according to any of claims 1 to 3, in which R₄ represents a group R₃ or a group of the formula -Q-B₄, Q being as defined in claim 1 and B₄ representing a group of the formula: 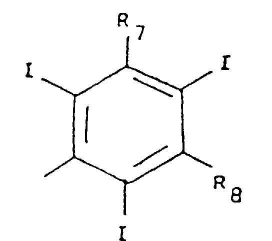 R₇ and R₈ having the same meaning as R₃.

8. Polyiodinated compounds according to any of claims I to 3, characterized in that it corresponds to the general formula V: 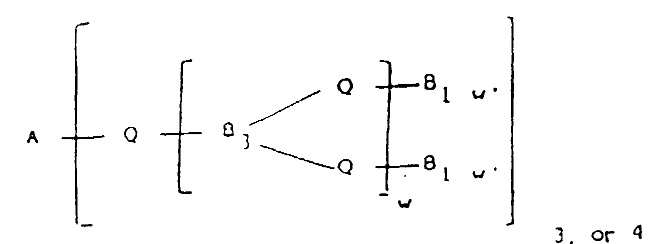 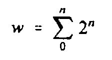 and w' = 2ⁿ (n representing an integer from 0 to 4) in which A is as defined in claim 1, Q represents the group 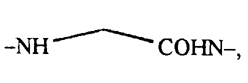 and B₃ represents the group: 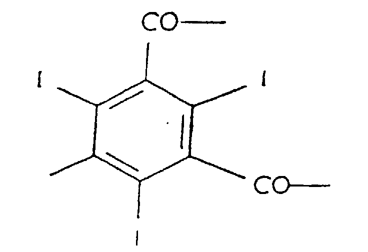 and B₁ represents the group 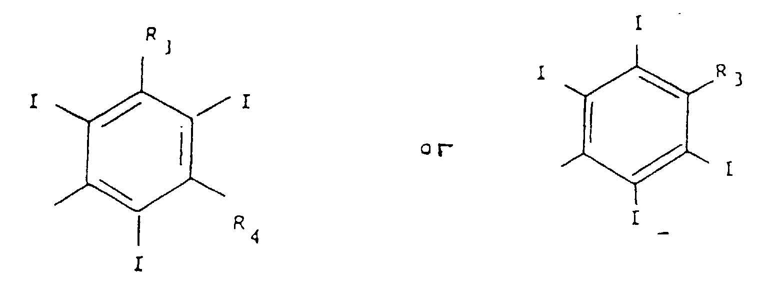 in which R₃ and R₄ are selected from CO⁻₂ M⁻ 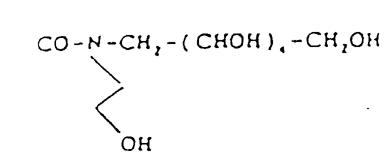 -CO-N(CH₂-(CHOH)₄-CH₂OH)₂

9. Polyiodinated compounds according to any of claims 1 to 3, characterized in that they are selected from: 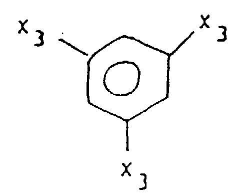 with:

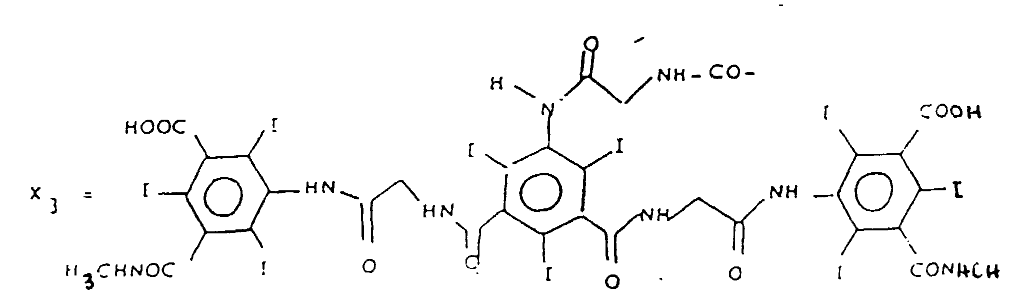

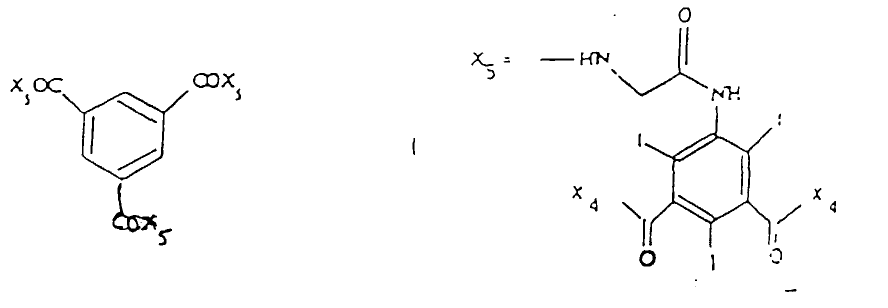

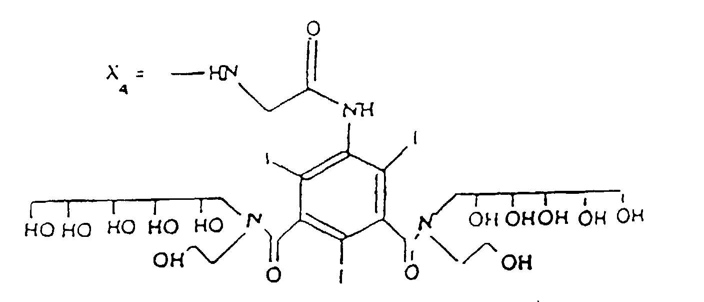

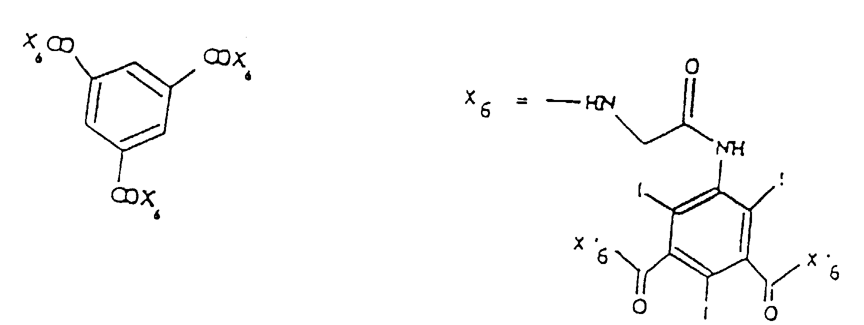

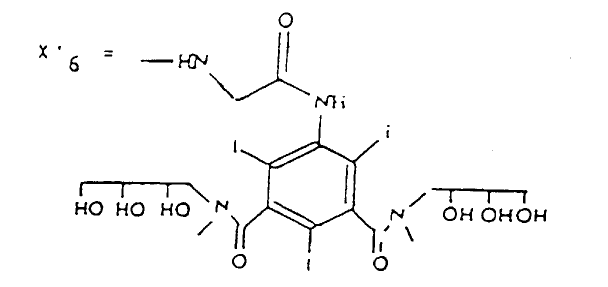

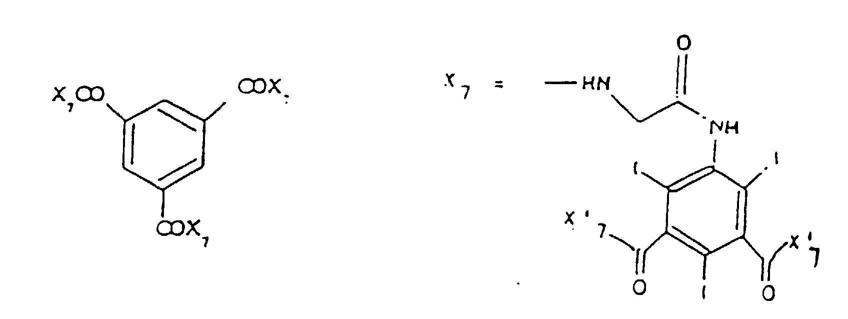

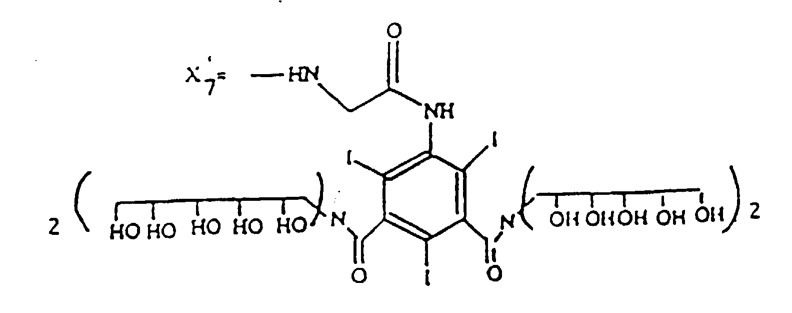

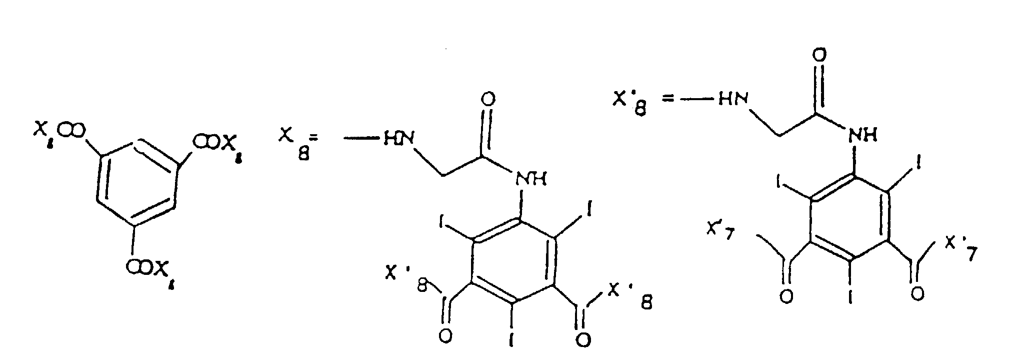

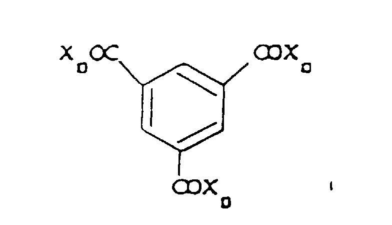

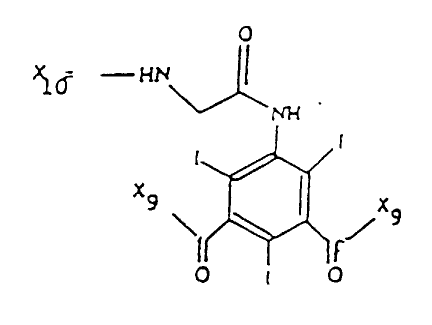

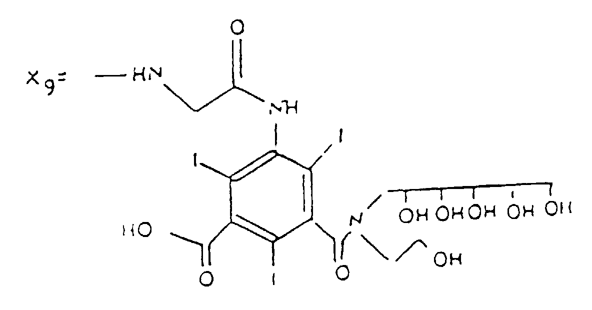

10. A contrast product characterized in that it contains at least one compound according to any of claims 1 to 9.

11. A contrast product according to claim 10, characterized in that it is formed by an aqueous solution of the compound or compounds.
